# EUROPEAN PATENT SPECIFICATION

(11) **EP 4 281 584 B1**
(45) Date of publication and mention of the grant of the patent: **08.04.2026**
(21) Application number: 22701025.3
(22) Date of filing: 20.01.2022
(51) Int. Cl.: C12Q 1/682

(54) **TARGET-DEPENDENT POLYMERISATION OF OLIGONUCLEOTIDES**
ZIELABHÄNGIGE POLYMERISATION VON OLIGONUKLEOTIDEN
POLYMÉRISATION D'OLIGONUCLÉOTIDES DÉPENDANT DE LA CIBLE

(30) Priority: 20.01.2021 GB 202100760
(43) Date of publication of application: 29.11.2023
(73) Proprietor: Qbiotix Limited, Ledbury HR8 1RZ (GB)
(72) Inventor: THRIPPLETON, Ian Peter, Bromsberrow Ledbury HR8 1RZ (GB)
(74) Representative: Stratagem IPM Limited
(86) International application number: PCT/GB2022/050153
(87) International publication number: WO 2022/157495

(56) References cited:
- EP-A1- 3 865 587
- WO-A1-2011/107606
- WO-A1-2015/118029
- WO-A1-2017/189525
- WO-A1-2018/044939
- WO-A1-2018/194755
- WO-A1-2018/217905
- WO-A1-2019/195346
- WO-A1-2020/099640
- WO-A1-2020/132527
- WO-A1-2020/249982
- WO-A2-2005/098049
- WO-A2-2021/221789
- CN-A- 109 762 875
- US-A1- 2019 292 580
- HARRY M. T. CHOI ET AL: "Next-Generation in Situ Hybridization Chain Reaction: Higher Gain, Lower Cost, Greater Durability", ACS NANO, vol. 8, no. 5, 27 May 2014 (2014-05-27), US, pages 4284 - 4294, XP055409053, ISSN: 1936-0851, DOI: 10.1021/nn405717p
- HARISH CHANDRAN ET AL: "An autonomously self-assembling dendritic DNA nanostructure for target DNA detection", BIOTECHNOLOGY JOURNAL, vol. 8, no. 2, 1 February 2013 (2013-02-01), pages 221 - 227, XP055203538, ISSN: 1860-6768, DOI: 10.1002/biot.201100499
- ZENG ZHUOER ET AL: "Nonlinear hybridization chain reaction-based functional DNA nanostructure assembly for biosensing, bioimaging applications", BIOSENSORS AND BIOELECTRONICS, ELSEVIER SCIENCE LTD, UK, AMSTERDAM , NL, vol. 173, 10 November 2020 (2020-11-10), XP086373199, ISSN: 0956-5663, [retrieved on 20201110], DOI: 10.1016/J.BIOS.2020.112814
- XU GAOLIAN ET AL: "Branched hybridization chain reaction-using highly dimensional DNA nanostructures for label-free, reagent-less, multiplexed molecular diagnostics", MICROSYSTEMS & NANOENGINEERING, vol. 5, no. 1, 1 December 2019 (2019-12-01), pages 37, XP055907828, Retrieved from the Internet <URL:https://www.nature.com/articles/s41378-019-0076-z.pdf> DOI: 10.1038/s41378-019-0076-z
- "2019-nCoV positive reference DNA fragment", GENESEQ,, 10 December 2020 (2020-12-10), XP002806115
- SAI BI ET AL: "Hyperbranched Hybridization Chain Reaction for Triggered Signal Amplification and Concatenated Logic Circuits", ANGEWANDTE CHEMIE INTERNATIONAL EDITION, vol. 54, no. 28, 26 May 2015 (2015-05-26), Hoboken, USA, pages 8144 - 8148, XP055554143, ISSN: 1433-7851, DOI: 10.1002/anie.201501457
- SAI BI ET AL: "Hyperbranched Hybridization Chain Reaction for Triggered Signal Amplification and Concatenated Logic Circuits", ANGEWANDTE CHEMIE, WILEY - V C H VERLAG GMBH & CO. KGAA, DE, vol. 127, no. 28, 26 May 2015 (2015-05-26), pages 8262 - 8266, XP071363861, ISSN: 0044-8249, DOI: 10.1002/ANGE.201501457
- YU CHUNMIAO ET AL: "Study on the Functionalization and Signaling Efficiency of the Hybridization Chain Reaction Using Traditional and Single Molecular Characterizations", ACS APPLIED BIO MATERIALS, vol. 4, no. 4, 19 April 2021 (2021-04-19), US, pages 3649 - 3657, XP055908029, ISSN: 2576-6422, Retrieved from the Internet <URL:https://pubs.acs.org/doi/pdf/10.1021/acsabm.1c00136> DOI: 10.1021/acsabm.1c00136
- PENG YIN ET AL: "Programming biomolecular self-assembly pathways", NATURE,, vol. 451, no. 7176, 17 January 2008 (2008-01-17), pages 318 - 322, XP002661061, DOI: 10.1038/NATURE06451
- TSUNEOKA YOUSUKE ET AL: "Modified in situ Hybridization Chain Reaction Using Short Hairpin DNAs", FRONTIERS IN MOLECULAR NEUROSCIENCE, vol. 13, 12 May 2020 (2020-05-12), XP055818695, DOI: 10.3389/fnmol.2020.00075
- HARRY M. T. CHOI ET AL: "Third-generation in situ hybridization chain reaction: multiplexed, quantitative, sensitive, versatile, robust", DEVELOPMENT, vol. 145, no. 12, 15 June 2018 (2018-06-15), GB, XP055590305, ISSN: 0950-1991, DOI: 10.1242/dev.165753
- GONG XUE ET AL: "Programmable intracellular DNA biocomputing circuits for reliable cell recognitions", CHEMICAL SCIENCE, vol. 10, no. 10, 6 March 2019 (2019-03-06), United Kingdom, pages 2989 - 2997, XP055908038, ISSN: 2041-6520, Retrieved from the Internet <URL:https://pubs.rsc.org/en/content/articlepdf/2019/sc/c8sc05217d> DOI: 10.1039/C8SC05217D
- DATABASE Geneseq [online] 10 December 2020 (2020-12-10), "2019-nCoV positive reference DNA fragment.", XP002806115, retrieved from EBI accession no. GSN:BIN00289 Database accession no. BIN00289
- ANG YAN SHAN ET AL: "Rational design of hybridization chain reaction monomers for robust signal amplification", CHEMICAL COMMUNICATIONS, vol. 52, no. 22, 1 January 2016 (2016-01-01), UK, pages 4219 - 4222, XP055908026, ISSN: 1359-7345, DOI: 10.1039/C5CC08907G
- REN KEWEI ET AL: "In Situ Genetically Cascaded Amplification for Imaging RNA Subcellular Locations", vol. 142, no. 6, 12 February 2020 (2020-02-12), pages 2968 - 2974, XP055843701, ISSN: 0002-7863, Retrieved from the Internet <URL:https://www.ncbi.nlm.nih.gov/pmc/articles/PMC7747016/pdf/nihms-1653424.pdf> DOI: 10.1021/jacs.9b11748

## Description

### Cross-reference to related application

The present application claims priority to GB patent application No. GB2100760.4, entitled "Target-dependent polymerization of oligonucleotides," filed January 20, 2021..

### Field of the invention

The present invention is directed to kits containing nucleic acid probes that may be used to detect a target nucleic acid or protein in a sample via non enzymatic amplification and to kits containing sets of amplification Chain Loop probes. The present invention also provides methods for detecting a target nucleic acid in a sample, methods of detecting an infectious agent in a sample.

### Sequence Listing

The instant application contains a Sequence Listing which has been filed electronically in ASCII format and is hereby incorporated by reference in its entirety. Said ASCII copy, created on January 19, 2022, is named TIP_SL.txt and is 6 KB in size.

### Background of the invention

The Hybridization Chain Reaction (HCR), Dirks and Pierce in 2004¹, discloses a detection system based on the reaction which consists of the isothermal assembly of stable DNA monomers in bigger structures at the addition of a specific initiator. The monomers in the original reaction design have hairpin-like secondary structures, composed of a long stem (18 nt), a short loop, and a short corresponding overhanging toehold (both 6 nt). The two species stably coexist in solution, thanks to their metastability, without any interaction in the experiment timescale.

The equilibrium of the system is altered by the initiator with a proper sequence: this triggers a cascade reaction of subsequent hybridisations, more precisely, a cascade of toehold-mediated strand displacements, leading to the formation of a long nicked double-stranded DNA. In absence of the initiator, the folded secondary structure of the monomers prevents the triggering of the reaction, since the toehold is closed within. The initiator activates the first hairpin, leading to the exposure of the short loop sequence, suitable for the binding to and opening of a second hairpin. The second hairpin, once incorporated, restarts the cycle with another copy of the first hairpin.

Srinivas et al look at closer details of the thermodynamics of HCR based on toehold principles. The free energy landscape in which toehold HCR is occurring operates at low to positive ΔG values².

Zeng et al.³ summarised the concepts and respective usefulness that are available for both linear and non-linear hybridization chain reactions for use in biosensing and bioimaging applications. "Hybridization chain reaction (HCR) can be divided into two categories: linear HCR and nonlinear HCR. In traditional linear HCR, the relatively slow kinetics and less sufficient sensitivity largely limit its scope of application. In the nonlinear HCR system, under the trigger of the initiator, the judicious designed substrate sequences (hairpin or hairpin-free) will self-assembly to dendritic or branched DNA nanostructures with exponential growth kinetics. Given the advantages of its enzyme-free, high-order growth kinetic, high sensitivity, and simple operation, nonlinear HCR is regarded as a powerful signal amplifier for the detection of biomarkers." However, Zeng clearly summarises the limitations of the concepts presented in the review. "First, the self-assembly of DNA nanostructures largely relies on kinetics and are driven by free energy, sometimes even in the absence of a catalyst. The reactions may proceed spontaneously, resulting in high background signals and false positive results. Therefore it is crucial to enhance the robustness of the reaction system and the priority of reactants in the chain formation. Second, Zeng claims the non-linear HCR is irreversible and hard to control. Consequently, it is difficult to monitor the change of reactant concentration over a long period of time. Moreover, the various shapes of amplification products make it rather difficult to achieve absolute concentration. Third, as an account of surface charge, size and biocompatibility, it is formidable to deliver nucleic acids directly into cells. Fourth, like traditional HCR, non-linear based biosensing technology is still at the laboratory level, because its poly-disperse assembly and the uncontrollable size of hybridisation product can only provide compromised reproducibility in complex sample matrix. Therefore it is a long way to go before volume production and rapid point-of-care-applications." "In spite of these challenges, we have to admit that non-linear HCR strategy is an innovation of isothermal amplification methods."

Yan Shan and Lin-Yue⁴ discuss guidelines for the sequence design of hairpin monomers in hybridization chain reaction (HCR) and Ren *et al⁵* report a novel genetically encoded *in situ* amplification method to noninvasively image the subcellular location of RNA targets in living cells.

In order to utilise HCR in a practical setting to avoid the need for skilled technicians and laboratories and to enable kits to be provided, where rapid, industrial, and routine applications become feasible a different approach to HCR is required. The present invention addresses these shortcomings.

### Brief summary of the invention

The present disclosure, at least in part, is based upon discovery of an improved manner of designing HCR oligonucleotide probes and performing HCR, specifically providing constructs that do not employ toe-holding and disclosing robust assays possessing high specificity that employ such constructs. Certain assays disclosed herein are useful for applications both in solution and *in situ,* with turnaround times of under two hours, preferably under one hour and more preferably in the order of 30 minutes, with hybridisation times of 1 to 15 minutes, preferably 3 to 12 minutes, typically about 5 to 10 minutes. Multifunctional hairpin loops are employed, where both the size of the loops and the length and sequence of the stem regions are designed to control both the specificity of hybridisation and control the free energy liberated upon hybridisation.

Thus, the present disclosure addresses the shortcomings in traditional HCR and provides a simple detection system that provides rapid, accurate results without the need for highly skilled work force and labs. The disclosure provides for a shortening of the hybridisation reaction time and involves steps amenable to automation.

Accordingly, the present disclosure provides in a first embodiment:
A kit for the detection of a target nucleic acid of interest; the kit comprising:
a) A hairpin Target Initiation probe and
b) A set of multiple hairpin Chain Loop probes to amplify a detectable signal arising from a signal generating component contained within the Chain Loop probe and/or the Target Initiation probe;
characterised in that:
the Target Initiation probe in its closed configuration is a hairpin nucleic acid comprising a complementary 3' and 5' stem sequence to form a double stranded stem portion and a single stranded target loop portion comprising a sequence complementary to the target nucleic acid;
further characterised in that a first Chain Loop probe comprises a hairpin loop structure with a sequence complementary to the 3' or 5' stem sequence of the Target Initiation probe and a signal generating component;
further characterised in that a second Chain Loop probe comprises a hairpin loop structure with a sequence complementary to the 3' or 5' stem sequence of the first Chain Loop probe and a signal generating component;
further characterised that subsequent Chain Loop probes comprise a hairpin loop structure with a sequence complementary to the 3' or 5' stem sequence of a prior Chain loop probe and a signal generating component;
wherein on binding of the target nucleic acid to the Target Initiation probe the stem region of the Target Initiation probe opens and permits hybridisation to the loop region of the first Chain Loop probe and causes sequential polymerisation of multiple Chain Loop probes to form a polymer capable of generating a signal; and
one of the Chain Loop probes comprises a hairpin loop structure with a further nucleic acid sequence extending from the 5' or 3' stem sequence which hybridises to the target loop of a prior Chain Loop probe, splitting the chain formation into two chains to form a dendrimer capable of generating a signal.

The signal generating component of the Chain Loop probes comprises one or more groups capable of generating a signal through fluorescence. In one embodiment the signal generating component of the Chain Loop probes comprises a Förster (Fluorescence) Resonance Energy Transfer (FRET) reporter group pair of donor and acceptor fluorophores such that in the closed position the acceptor and donor fluorophore are separated to prevent resonance transfer and generation of a detectable signal. In one embodiment the signal may be generated through time resolved FRET (TR-FRET), wherein Lanthanides are complexed via a complexing agent such as DOTA or DOPA, wherein the complexing agent is linked to an oligonucleotide.

In another embodiment, the signal generating component of the Chain Loop probes may comprise one or more groups capable of generating a signal through chemiluminescence. A list of non-limiting examples includes biotinylation followed by detection with Streptavidin-labelled with a signalling enzyme or fluorophore, hapten-labelling followed by detection using hapten-specific labelled antibodies, or use of radiolabelled oligonucleotides with direct scintillation counting or via scintillation proximity assays.

Further, in any embodiment, the Target Initiation probe may comprise a signal generating component capable of generating a signal through fluorescence or chemiluminescence, as described for Chain Loop probes.

In an aspect of the disclosure the final Chain Loop probe comprises a nucleic acid sequence in its stem that is complementary to a sequence of a preceding set of Chain Loop probes.

The present disclosure further provides kits wherein the Gibbs free energy of binding of the Target Initiation probe and of one or more Chain Loop probes is negative under respective hybridisation conditions and the Tₘ of the stem formation is higher than the effective hybridising temperature of the Chain Loop probes.

Preferably there are at least 3, 4, 5, 6, or more different Chain Loop probes provided in a kit.

The Chain Loop probes in one embodiment, carry a target loop with a unique sequence.

In a preferred embodiment there is provided a kit, wherein the 5' or the 3' stem forming sequence of the Target Initiation probe and Chain Loop probe thermodynamically favours hybridisation with a complementary sequence over the base-pairing of its respective hairpin loop, and wherein the Gibbs free energy of stem formation of each probe is less negative than the Gibbs free energy of hybridisation with a target nucleic acid sequence.

In an embodiment the reporter pair generates a detectable signal only when a Chain Loop probe (n) binds to a Chain Loop probe (n-1) and the hybrid brings the donor and acceptor fluorophores close enough to enable Förster (Fluorescence) Resonance Energy Transfer (FRET) or for a pair or a chemical crosslinking group(s) to form a chemical cross-link. In an alternative embodiment, the Chain Loop probes carry biotin moieties as binding points for biotin-streptavidin detection systems.

In an embodiment the Target Initiation probe hybridises at a hybridisation temperature of 5, 7.5, 10, 12.5, 15, 17.5 ºC higher than the Chain Loop probes.

The Kits of the disclosure can be utilised for multiplexing. Thus in an embodiment two or more Target Initiation probes and two or more sets of Chain Loop probes are provided for simultaneous reporting on the presence or absence of two or more nucleic acids. Alternatively kits are provided which have two or more Target Initiation probes directed to discrete sequences on the same nucleic acid target and a single set of Chain Loop probes to enhance the reporting on the presence or absence of the target nucleic acid.

In an aspect of the disclosure there is provided a method for the detection of a target nucleic acid in a sample comprising:
a). performing a hybridisation reaction reacting a Target Initiation probe with a sample,
b). performing a hybridisation reaction reacting the product of step a) with a set of Chain Loop probes wherein on binding of the target nucleic acid to the Target Initiation probe the stem region of the Target Initiation probe opens and permits hybridisation to the loop region of the first Chain Loop probe and causes sequential polymerisation of the set of Chain Loop probes to form a polymer or dendrimer capable of generating a signal;
c). detecting the presence or absence of a signal that indicates the presence or absence of the target nucleic acid
characterised in that:
the Target Initiation probe in its closed configuration is a hairpin nucleic acid comprising complementary 3' and 5' stem sequences to form a double stranded stem portion and a single stranded target loop portion comprising a sequence complementary to the target nucleic acid;
further characterised in that a first Chain Loop probe comprises a hairpin loop structure with a sequence in the loop complementary to the 5' or 3' stem sequence of the Target Initiation probe and a signal generating component;
further characterised in that a second Chain Loop probe comprises a hairpin loop structure with a sequence in the loop complementary to the 5' or 3' stem sequence of the first Chain loop probe and a signal generating component;
further characterised that subsequent Chain Loop probes comprise a hairpin loop structures with a sequence complementary to the 5' or 3' stem sequence of a prior Chain loop probe and a signal generating component;
wherein on binding of the target nucleic acid to the Target Initiation probe the stem region of the Target Initiation probe opens and permits hybridisation to the loop region of the first Chain Loop probe and causes sequential polymerisation of multiple Chain Loop probes to form a polymer capable of generating a signal; and wherein one of the Chain Loop probes comprises a hairpin loop structure with a further nucleic acid sequence extending from the 5' or 3' stem sequence which hybridises to the target loop of another Chain Loop probe splitting the chain formation into two chains to form a dendrimer capable of generating a signal.

Preferably, the hybridising step a) is carried out at a temperature of 5, 7.5, 10, 12.5, 15, or 17.5 ºC higher than the hybridisation reaction of step b).

In an embodiment of the method of the disclosure the reaction of step a) and/or step b) is carried out in the presence of formamide in the hybridising buffer.

In an embodiment of the method of the disclosure, the hybridisation of step a) is carried out at 50ºC in the presence of 20% formamide and/or step b) is carried out at 30ºC in the presence of 20% formamide.

In an embodiment of the method of the disclosure, the hybridisation of step a) is carried out at 62.5ºC equivalent and/or step b) is carried out at 50ºC equivalent.

In another embodiment the present disclosure provides for the detection of two or more nucleic acid sequences in a sample, wherein step a) above, comprises two or more sets of Target Initiation probes and step b) above, comprises two or more sets of Chain Loop probes capable of reporting on the presence or absence of two or more target nucleic acid sequences. Alternatively, the disclosure provides a method for the detection of a nucleic acid target wherein step a) two or more Target Initiation probes are provided which hybridise to discrete sequences on the same target nucleic acid and step b) comprises a single set of Chain Loop probes. The disclosure further provides a sample formulation for use with kits and methods of the disclosure herein described comprising an aqueous sample to be tested the sample formulation comprising:
one or more chaotropic agents, one or more polyols as a solvent which complexes water molecules, a reducing agent which solubilises mucus and a compound which neutralises the negatively charged phosphate backbone of any nucleic acid. Preferably the chaotropic agent is Guanidine Hydrochloride, the reducing agent is Tris(2-carboxyethyl) phosphine hydrochloride (TCEP), the polyol is Glycerol, and the compounds which neutralise the negatively charged phosphate backbone of any nucleic acid comprise Mg²⁺, Spermidine, or Poly-Lysine or mixtures thereof.

Another aspect of the instant disclosure provides a composition that includes one or more sequences disclosed herein (SEQ ID NOs: 1-25). In one embodiment, the instant disclosure provides SEQ ID NOs: 21-25 as a set of hairpin loop probe sequences. In alternative embodiments, SEQ ID NOs: 5-10 or SEQ ID NOs: 15-20 are provided as a set, respectively.

A related aspect of the instant disclosure provides a kit that includes one or more of SEQ ID NOs: 1-25 disclosed herein, and instructions for its use.

A further aspect of the instant disclosure provides a method for selecting sequences for a set of four or more hairpin loop nucleic acid probes capable of hybridizing linearly or exponentially with one another in the presence of a target sequence, the method involving identifying candidate sequences for a first hairpin loop probe, a second hairpin loop probe, a third hairpin loop probe, a fourth hairpin loop probe, and optionally successive additional hairpin loop probes, where each of the first hairpin loop probe, the second hairpin loop probe, the third hairpin loop probe, the fourth hairpin loop probe, and the optionally successive additional hairpin loop probes (i) possesses a hairpin structure, where the probe comprises 5'-terminal and 3'-terminal complementary, annealed stem loop sequence regions, optionally including a terminal overhang between 5' and 3' termini, and a single-stranded hairpin loop sequence that joins the annealed stem sequence regions (at minimum when in a solution lacking other nucleic acid sequences), where the annealed stem sequences of each hairpin loop are selected to possess a higher Gibbs free energy (ΔG) value in solution for the intra-molecule stem loop structure formation than the ΔG value for inter-molecule annealing between the single-stranded hairpin loop sequence and its target sequence in a distinct oligonucleotide (i.e., the distinct oligonucleotide being an initiator target sequence or a complementary sequence within a stem loop of a distinct hairpin probe); and (ii) possesses a 5'- or 3'-terminal first fluorophore modification and a second fluorophore modification at an internal nucleotide residue position adjacent to or within the single-stranded hairpin loop sequence; where thermodynamic properties including Gibbs free energy (ΔG) values of the candidate sequences are predicted or evaluated to identify a set of four or more hairpin loop nucleic acid probe sequences where: the single-stranded hairpin loop sequence of the first hairpin loop probe is complementary to a target sequence not found within the set of hairpin loop probe sequences, where the single-stranded hairpin loop sequence of the first hairpin loop probe is capable of annealing to the target sequence with a ΔG value that is lower than the ΔG value for intra-molecule first hairpin loop probe stem loop structure formation; the single-stranded hairpin loop sequence of the second hairpin loop probe is complementary to at least one stem region sequence of the first hairpin loop probe and the single-stranded hairpin loop sequence of the second hairpin loop probe is capable of annealing to the complementary stem region sequence of the first hairpin loop probe with a ΔG value that is lower than the ΔG value for intra-molecule second hairpin loop probe stem loop structure formation; the single-stranded hairpin loop sequence of the third hairpin loop probe is complementary to at least one stem region sequence of the second hairpin loop probe and the single-stranded hairpin loop sequence of the third hairpin loop probe is capable of annealing to the complementary stem region sequence of the second hairpin loop probe with a ΔG value that is lower than the ΔG value for intra-molecule third hairpin loop probe stem loop structure formation; and the single-stranded hairpin loop sequence of the fourth hairpin loop probe is complementary to at least one stem region sequence of the third hairpin loop probe and the single-stranded hairpin loop sequence of the fourth hairpin loop probe is capable of annealing to the complementary stem region sequence of the third hairpin loop probe with a ΔG value that is lower than the ΔG value for intra-molecule fourth hairpin loop probe stem loop structure formation, thereby selecting sequences for a set of four or more hairpin loop nucleic acid probes capable of hybridizing linearly or exponentially with one another in the presence of a target sequence.

In certain embodiments, each of the first hairpin loop probe, the second hairpin loop probe, the third hairpin loop probe, the fourth hairpin loop probe, and the optionally successive additional hairpin loop probes possesses a total length of 120 nucleotides or less. Optionally, each probe possesses a total length of 100 nucleotides or less. Optionally, each probe possesses a total length of 90 nucleotides or less. Optionally, each probe possesses a total length of 80 nucleotides or less. Optionally, each probe possesses a total length of 70 nucleotides or less.

In some embodiments, the hairpin sequence of the first hairpin loop probe is capable of annealing to the target sequence with a ΔG of at least about -10 kcal/mol ± 1.5kcal/mol; the hairpin sequence of the second hairpin loop probe is capable of annealing to the complementary stem region sequence of the first hairpin loop probe with a ΔG of at least about -10 kcal/mol ± 1.5kcal/mol; the hairpin sequence of the third hairpin loop probe is capable of annealing to the complementary stem region sequence of the second hairpin loop probe with a ΔG of at least about -10 kcal/mol ± 1.5kcal/mol; the hairpin sequence of the fourth hairpin loop probe is capable of annealing to the complementary stem region sequence of the third hairpin loop probe with a ΔG of at least about -10 kcal/mol ± 1.5kcal/mol; and/or the hairpin sequence of the (n+1)^{th} hairpin loop probe is capable of annealing to the complementary stem region sequence of the n^{th} hairpin loop probe with a ΔG of at least about -10 kcal/mol ± 1.5kcal/mol.

In one embodiment the method further includes synthesizing the set of four or more hairpin loop nucleic acid probes selected, thereby selecting and preparing sequences for the set.

In another embodiment, at least one of the hairpin loop probes includes complementary, annealed stem loop sequence regions where each strand of the stem loop sequence region is capable of being hybridised by a distinct single-stranded hairpin loop sequence of a different hairpin loop probe of the set of four or more hairpin loop nucleic acid probes, thereby forming a branch structure during polymerisation/progression of inter-molecular hybridisation events between hairpin loop probes.

In each hybridisation step there is liberated sufficient free energy to secure specificity of the hybridisation as well as sufficient energy to ensure spontaneity and therefore speed of the assay.

### Brief description of the drawings

The following detailed description, given by way of example, but not intended to limit the disclosure solely to the specific embodiments described, may best be understood in conjunction with the accompanying drawings, in which:
***FIG. 1*** shows a schematic of Target Initiation probe and Chain Loop probe design.
***FIG. 2*** shows a schematic of a target nucleic acid (here, a target nucleic acid sequence of a virus) capture and detection process.
***FIG. 3*** shows a schematic of a Target Initiation probe annealing to a target nucleic acid sequence, thereby destabilizing and disrupting the Target Initiation probe stem loop otherwise formed and favoured in the absence of the nucleic acid target sequence.

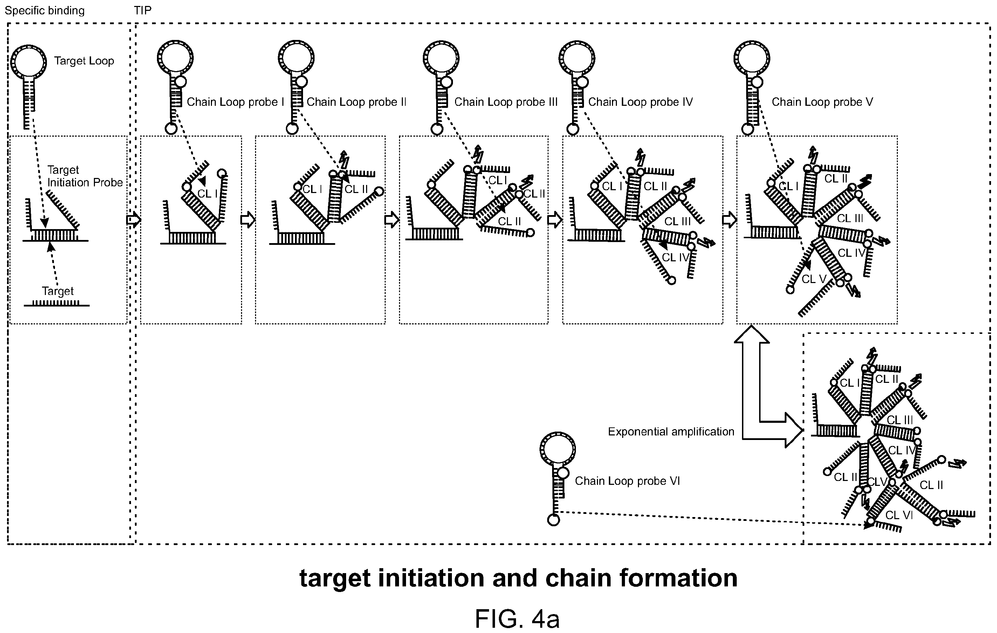

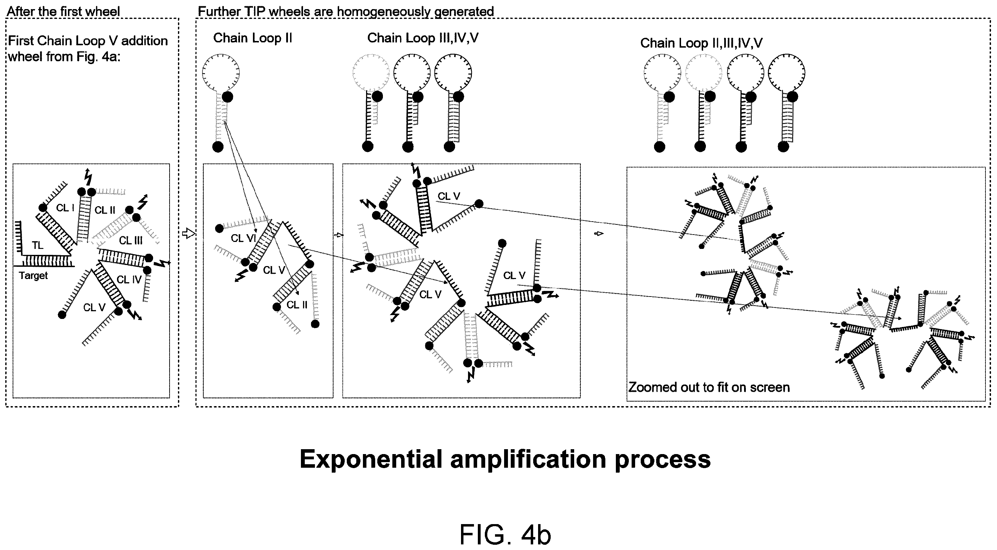

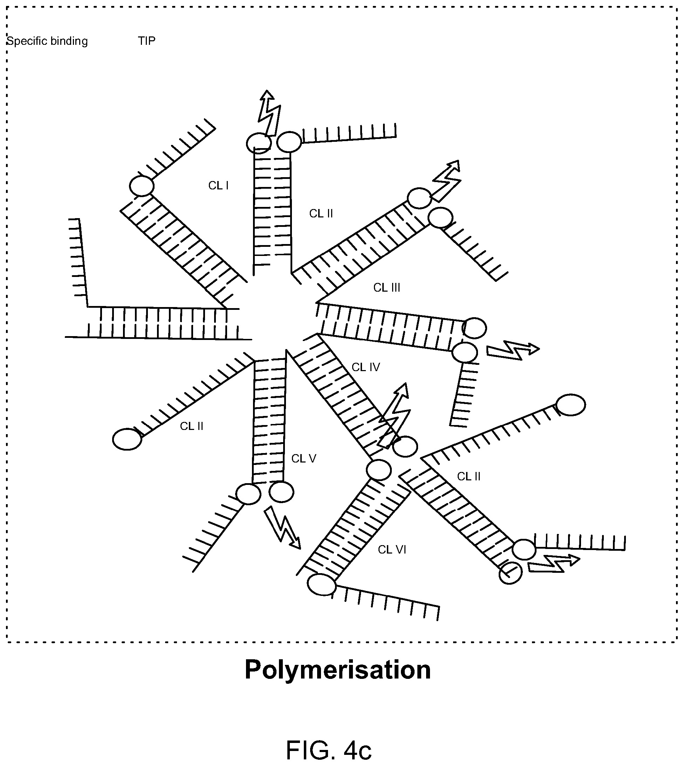

***FIGs. 4a to 4c*** show schematics depicting a target recognition and signal amplification process of the instant disclosure, termed Target Initiated Polymerisation ("TIP"). ***FIG. 4a*** shows a schematic of target recognition occurring via specific annealing of a target nucleic acid sequence by a target sequence-recognizing Target Loop hairpin nucleic acid structure, which results in disruption of the stem loop structure of the Target Loop hairpin upon target nucleic acid binding and exposure of Target Loop stem sequences otherwise masked via stem annealing in the absence of target nucleic acid. Successive Chain Loop probes (here, Chain Loop probes I, II, III, IV, V, and VI) each respectively possess a terminal fluorophore and an intra-molecular fluorophore, positioned at a distance designed to limit intra-molecular fluorophore-fluorophore interaction. Upon binding of a newly-exposed stem loop of the Target Initiation probe in the presence of target nucleic acid, disruption of the fluorophore-labeled Chain Loop I stem reveals a stem region sequence that is then annealed by the loop sequence of Chain Loop II, resulting in an inter-molecular pairing of fluorophores (here, a terminal flurophore of Chain Loop I and an internal fluorophore of Chain Loop II), producing a signal (here, via fluorescence resonance energy transfer (FRET)). Loop regions of successive Chain Loop probes (Chain Loop n+1 probes) subsequently anneal to newly-revealed stem loop sequences of preceding Chain Loop n probes, producing further inter-molecular pairs of fluorescent moieties and progressively amplified signaling, as an initial "wheel" forms upon binding of the Chain Loop V loop sequence to a newly unmasked stem sequence in Chain Loop IV. In the instant schematic, because the two complementary stem sequences of Chain Loop V are designed such that one stem sequence of Chain Loop V is capable of being specifically bound by the loop sequence of Chain Loop VI while the other stem sequence of Chain Loop V is capable of being specifically bound by the loop sequence of Chain Loop II, Chain Loop V thereby introduces a branch point in the growing chain and is termed a "splitter probe" as detailed below. A stem sequence of Chain Loop V-annealed Chain Loop VI is then capable of being recognized and bound by the Chain II loop sequence, thereby promoting further chains of annealing events and signal amplification on both sides of the splitter probe (here, Chain Loop V). Introduction of such splits allows for exponential increases in [Chain Loop n+1 loop sequence-to-Chain Loop n stem sequence] binding events to proceed, with accompanying exponential amplification of inter-molecular fluorophore-fluorophore signalling. ***FIG. 4b*** shows a schematic that provides additional detail regarding Chain Loop probe interactions during the exponential growth phase depicted. ***FIG. 4c*** shows an even more detailed schematic of Chain Loop probe interactions in which Chain Loop V introduces branching. Target Initiated Polymerisation (TIP) as disclosed herein therefore produces a homogeneous amplification chain. Each Chain Loop has a unique stem sequence to specifically bind to the previous Chain Loop. The Chain Loops are dispensed as an Amplification Mix which isocratically feeds the growing amplification chain. Because Chain Loop I and V carry identical stem sequences, additional rounds of hybridisation-mediated polymerization can proceed, with exponential expansion promoted via introduction of splitter probe (e.g., Chain Loop V as exemplified in certain aspects herein). New viruses (or other target sequences of interest) can be detected simply by changing the first Target Loop sequence.
***FIG.** 5* shows a diagram of the dendrimer-type structure produced by successive binding of Chain Loop probes, with Chain Loop V being a splitter probe that introduces branching and therefore allows for exponential chain extension and signal amplification, as described elsewhere herein (the sequential Chain Loop extension process repeats itself, generating additional FRET signals and therefore quickly amplifying the total signal indicating detection of the target nucleic acid sequence.
***FIG. 6*** provides a flow chart showing successive steps and projected timing of an exemplary assay procedure of the instant disclosure.
***FIG. 7*** shows kinetics of polymerization events as disclosed herein, noting that peak fluorescent signal was observed at 10 min.
***FIG. 8*** shows that the exponential development of TIP signal is modelled to outpace quantitative PCR signal development, in view of the increased introduction of fluorophores per cycle in TIP to extending chains to produce FRET signal, as compared to quantitative PCR introduction of a single molecular beacon per amplicon per cycle.
***FIG. 9*** shows calculated thermodynamic values for each of the Chain Loop probes of the exemplified set of the instant disclosure comprising six discrete Chain Loop probe sequences, which demonstrates dramatic differences in such values between two state inter-molecular binding conditions and single-state intra-molecular binding conditions, for each respective Chain Loop sequence examined/modeled.
***FIG. 10*** shows a flowchart of the TIP process employed herein to assess levels of SARS-CoV-2 target nucleic acid sequence in solution, using the set of five Chain Loop probe sequences exemplified herein.

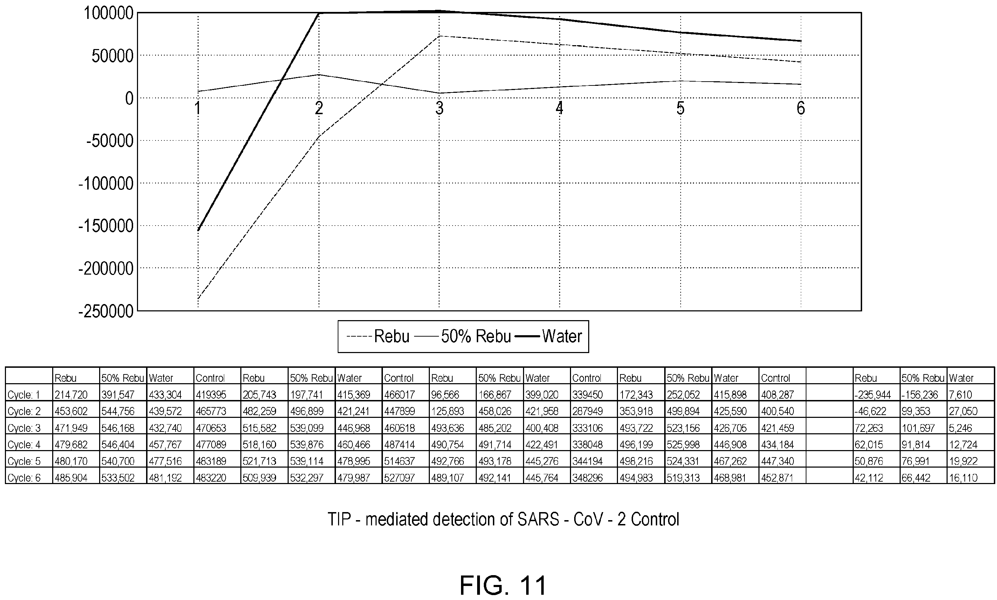

***FIG. 11*** shows that the TIP process, here employing a set of five Chain Loop probe sequences presently disclosed, was able to detect with rapidly amplified signal a SARS-CoV-2 viral RNA comprising a target sequence recognized by the Target Initiation probe of the TIP process, when the viral target RNA was either directly formulated in microbial release buffer ("ReBu", which is a sputum liquefier, RNA extraction and transport medium) or was first formulated in water and then mixed with an equal proportion of ReBu ("50% ReBu", which was employed as reflecting the final assay, where a volume of a test sample, *e.g*., waste water or saliva, is added to 1 mL of ReBu in a 2 mL screw-cap vial, and is then assayed). Both viral target RNA-containing samples ReBu and 50% ReBu exhibited rapid signal production at levels over the water only (no target RNA) control, noting that fluorescent signal levels are shown on the y-axis while plate reading cycles of approximately 65 seconds each are shown on the x-axis. Repeated reading cycles were performed to ensure that signal reached a plateau value, and interestingly, the greatest nucleic acid detection signal levels over background were obtained in the approximately 2-3 minute timeframe, after which a gradual decay in RNA-detecting signal levels over control (no nucleic acid and/or background levels) occurred.
***FIG. 12*** shows a dose response curve obtained using the TIP process disclosed herein upon a SARS-CoV-2 RNA dilution series in RNase free water. Associated tables are also shown. The dose response curve demonstrates that TIP-mediated detection of target RNA (here, SARS-CoV-2) was both rapid and quantitative.

### Detailed description of the invention

### Definitions:

### Capture Probe

In the present disclosure a Capture probe comprises a nucleic acid bound to a particle whereby said Capture probe is able to hybridise a complementary target nucleic acid or protein of interest. Such particle-based Capture probes are well known in the literature, and are commonly used to select and concentrate target nucleic acids or proteins within a solution. A variety of particles are readily available commercially including magnetic beads, Sepharose particles (both magnetic, ones carrying a binding protein such as but not limited to avidin derivatives, antibodies, lectins, or chemically activated), and beads with chemical cross-linkers. In a preferred embodiment magnetic Sepharose-based particles are used to concentrate the nucleic acid of interest within a sample.

### Hairpin Loop

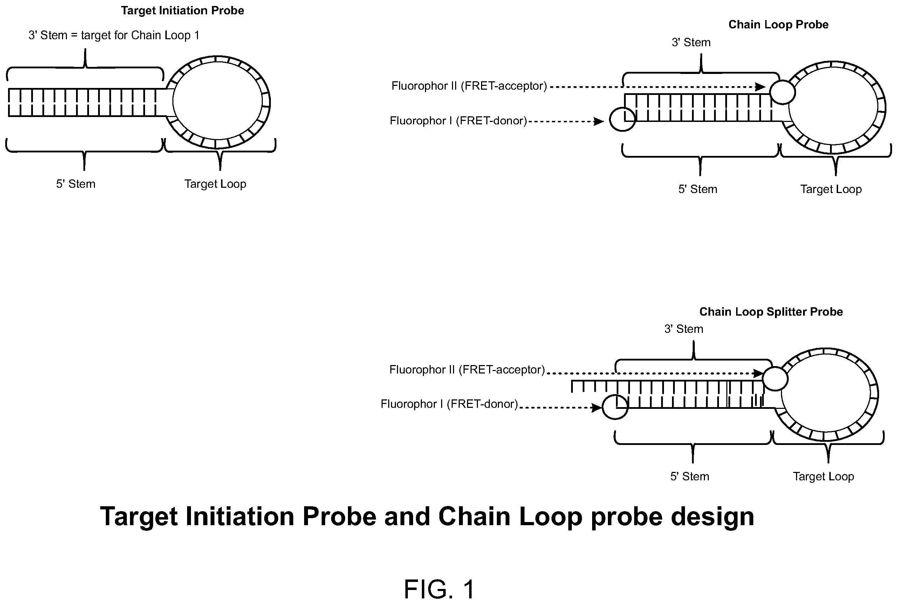

A hairpin loop, also known as a stem loop, is the intramolecular base pairing of a single-stranded nucleic acid with complementary sequences when read in opposite directions such that it adopts a distinct structure comprising a stem and an intermittent single stranded nucleotide sequence forming a non-base paired loop (FIG. 1).
a) Base pairing
   The stem sequence of a hairpin loop comprises a 5' stem and a 3' stem with sufficient complementarity between the respective nucleic acid sequences to facilitate intramolecular base pairing (under hybridisation conditions), where the respective action is referred to as 'base pairing'.
b) Hybridisation
   Intermolecular base pairing of two separate and independent nucleotide sequences. The respective action is referred to as the hybridising of the loop section to a separate oligonucleotide, in short 'hybridising'.

### Hybridisation conditions

The temperature required to achieve a specific hybridisation within a designed step in the sequence of events together with for example, mono- and multivalent salt, formamide, and detergent concentrations as shown in (Table 4a).

### Thermodynamically Preferred

The term thermodynamically preferred encompasses the term entropy (S), enthalpy (H) and melting temperature (Tₘ), which are used in the description of base-pairing and hybridisation processes. All three are integrated in the calculation of the respective thermodynamic potential generated in the processes in the form of free (Gibbs) energy. The Gibbs free energy is defined as: ΔG = ΔH - Tₘ ΔS. In order for a reaction to take place spontaneously the ΔG value needs to be negative. A positive ΔG requires energy to be infused for a reaction to take place.

### Target Initiation Probe

In the present disclosure, a Target Initiation probe comprises a 5' stem sequence, a target loop and a 3' stem sequence. The Target Initiation probe can adopt an open or closed conformation, herein referred to as an open Target Initiation probe or closed Target Initiation probe respectively. A closed Target Initiation probe comprises a hairpin loop, such that the 5' and 3' stem sequences of the probe are complementary and follow Watson-Crick base pairing rules to form helical stems, whilst the intervening sequence forms a single stranded target loop. In the closed conformation, the 5' and 3' stem sequences are masked with respect to any oligonucleotide competing for either sequence. Both loop and stem sequences interact with each other via base stacking forces, influencing the three dimensional structure of a hairpin loop and respective thermodynamic properties.

The target loop of the Target Initiation probe is designed to have a sequence which is complementary to a target nucleic acid of interest. In the open conformation, the 5' and 3' stem sequences are exposed and capable of interacting with other complementary nucleic acid sequences. In the absence of a target nucleic acid, it is thermodynamically favourable for the Target Initiation probe to adopt a closed conformation. If the target nucleic acid is available and under hybridisation conditions, the target loop of the Target Initiation probe hybridises with this sequence, causing the Target Initiation probe to change from a closed to an open conformation, unmasking the 5' and 3' stem sequences. The 5' and 3' stem sequences of the open Target Initiation probe can act as targets for Chain Loop probes, as described below.

The thermodynamic properties of the Target Initiation probe are essential for its function. While initially maintaining the hairpin loop structure, under hybridisation conditions the Target Initiation probe will unfold as specific hybridisation takes place. To maintain the initial formation, the helical stem has a negative Gibbs free energy value (ΔG) under hybridisation conditions. It is well known in the art that hairpin loops have a higher specificity than respective linear oligonucleotides. In order to drive target hybridisation, the ΔG of the target loop sequence hybridising to a complementary target sequence is more negative than the ΔG of helical stem formation. The higher the difference in ΔG, the faster a hybridisation will take place. Therefore, in the absence of a target nucleic acid, the Target Initiation probe adopts a closed conformation. However, in the presence of a target nucleic acid and under hybridisation conditions it is thermodynamically favourable for the Target Initiation probe to spontaneously hybridise with the target nucleic acid, and adopt an open conformation. Therefore, only in the presence of a target nucleic acid will the 5' and 3' stem sequences of the Target Initiation probe be unmasked, allowing subsequent polymerisation, as described below.

### Chain Loop Probe

In the present disclosure a Chain Loop probe comprises a 5' stem sequence, a non-base-paired target loop and a 3'stem sequence. In analogy to the Target Initiation probe, the Chain Loop probe can also adopt an open or closed conformation following the same base-pairing principles, herein referred to as an open Chain Loop probe or closed Chain Loop probe respectively. A closed Chain Loop probe comprises a hairpin loop, such that the 5' and 3' stem sequences of the probe are base-paired and therefore masked, and the intervening sequence forms a single stranded target loop. The target loop of the Chain Loop probe (n) is designed to have a sequence which is complementary to a target nucleic acid. In a preferred embodiment, the target nucleic acid is the 5' or 3' stem sequence of Chain Loop probe (n-1), or the 5' or 3' stem sequence of a Target Initiation probe, or the 5' or 3' stem sequence of the splitter probe. In the open Chain Loop probe, the 5' and 3' stem sequences are exposed and capable of interacting with other complementary nucleic acid sequences. In the absence of a target nucleic acid, it is thermodynamically favourable for the Chain Loop probe to adopt a closed conformation. If the target nucleic acid is available, the target loop of the Chain Loop probe can hybridise with this sequence, causing the Chain Loop probe to change from a closed to an open conformation, unmasking the 5' and 3' stem sequences. The 5' and 3' stem sequences of the open Chain Loop probe can act as a target for subsequent chain loop probes, leading to formation of a polymer.

The Chain Loop probes may contain a Förster (Fluorescence) Resonance Energy Transfer (FRET) reporter pair, whereby in the closed position the donor fluorophore and acceptor fluorophore are sufficiently separated such that resonance transfer is inhibited by distance and a signal is not generated. Upon hybridisation, the acceptor fluorophore of open Chain Loop probe (n) and the donor fluorophore of open Chain Loop probe (n-1) are sufficiently close for resonance to take place and to generate a signal. It can be understood by a person skilled in the art that Chain Loop Probe (n) may provide the donor fluorophore, whilst chain loop probe (n-1) may provide the acceptor fluorophore or vice versa.

The thermodynamic properties of the Chain Loop probe are essential for its function. While initially maintaining the hairpin loop structure, under hybridisation conditions the Chain Loop probe will unfold as specific hybridisation takes place. To maintain the initial formation, the helical stem has a negative Gibbs free energy value (ΔG) under hybridisation conditions. It is well known in the art that hairpin loops have a higher specificity than respective linear oligonucleotides. In order to drive target hybridisation, the ΔG of the target loop sequence hybridising to a complementary target sequence is more negative than the ΔG of helical stem formation. The higher the difference in ΔG, the faster a hybridisation will take place. Therefore, in the absence of a target nucleic acid, the Chain Loop probe adopts a closed conformation. However, in the presence of a target nucleic acid and under hybridisation conditions it is thermodynamically favourable for the Chain Loop probe to spontaneously hybridise with the target nucleic acid, adopting an open conformation.

The Target Initiation and Chain Loop probes of the instant disclosure can be of any length capable of forming a hairpin structure, including 80 nucleotides or more, 100 nucleotides or more, 120 nucleotides or more, 150 nucleotides or more, or even 200 nucleotides or more. In certain embodiments, probes of the instant disclosure have a total length of 200 nucleotides or less, optionally 150 nucleotides or less, optionally 120 nucleotides or less, optionally 100 nucleotides or less, optionally 90 nucleotides or less, optionally 80 nucleotides or less, and optionally 70 nucleotides or less.

### Splitter Probe

In the present disclosure a Splitter probe comprises a 5' stem sequence, a non-base-paired target loop sequence and a 3'stem sequence as defined for Chain Loop probes. The Splitter probe can also adopt an open or closed conformation, herein referred to as an open Splitter probe or closed Splitter probe respectively. A closed Splitter probe comprises a hairpin loop, such that the 5' and 3' stem sequences of the probe are base-paired and therefore masked, and the intervening sequence forms a single stranded target loop. The target loop of the splitter probe can be designed to have a sequence which is complementary to a target nucleic acid. In a preferred embodiment, the target nucleic acid is the 5' or 3' stem sequence of a Chain Loop probe. In the open Splitter probe, the 5' and 3' stem sequences are exposed and capable of interacting with other complementary nucleic acid sequences. In the absence of a target nucleic acid, it is thermodynamically favourable for the Splitter probe to adopt a closed conformation. If the target nucleic acid is available, the target loop sequence of the Splitter probe can hybridise with this sequence, causing the splitter probe to change from a closed to an open conformation, unmasking the 5' and 3' stem sequences. Both the 3' **and** 5' stem sequences of the open splitter probe can act as a target for subsequent chain loop probes, leading to formation of a branched polymer. The Splitter probe differs from a Chain Loop probe in that the 5' or 3' stem is elongated providing a distinguished sequence to which a further unique Chain Loop probe can hybridise; carrying the same 5' stem sequence of any other Chain Loop probes. Thus, the Splitter Probe is able to simultaneously hybridise two chain loops and initiated the formation of a new chain while continuing the elongation of the initial chain.

The splitter probes may contain a Förster (Fluorescence) Resonance Energy Transfer (FRET) reporter group pair, whereby in the closed position the donor fluorophore and acceptor fluorophore are sufficiently separated such that a signal is not generated. Upon hybridisation, the acceptor fluorophore of the splitter probe and the donor fluorophore of a hybridised open chain loop probe are sufficiently close to generate a signal. It can be understood by a person skilled in the art that splitter probe may provide the donor fluorophore, whilst chain loop probe may provide the acceptor fluorophore.

### Detection of nucleic acids

The present disclosure provides a method of detecting a target nucleic acid in a sample. This method may be employed, for example, in the detection of the presence of an infection with a pathogenic organism in a patient where the target nucleic acid in the sample is derived from an infectious agent which causes the infection in a patient. Alternatively, the target nucleic acid may be a cancer gene marker. Alternatively, the sample may be derived from food sources, veterinary or water sources, pharmaceutical laboratories and manufacturing plants where there is a need to check for biological contamination.

In certain embodiments the target nucleic acid may be from a bacterium, virus, fungus or parasite. In some embodiments the nucleic acid is from Influenza virus, including Influenza A or B, or a coronavirus, including SARS causing coronavirus such as SARS-CoV-2 or SARS-CoV-1.

The sample material may be from bodily fluids, blood, sputa, saliva, or faeces or may be derived from biopsies from for example, tumour or ulcer samples. In an embodiment, the sample on which the method of the disclosure is conducted, is a saliva sample, readily obtainable from a person of any age. In order to produce samples from very large cohorts including individuals the procedure needs to be simple, safe and not requiring trained personnel. Small volumes of saliva may be produced even by children without distress which is more effective than obtaining samples from the upper respiratory tract by the use of swabs. Saliva samples may be obtained by gargling with a few millilitres of saliva inducing fluid to readily produce a copious saliva sample, or by sucking on a sponge. The saliva may be transferred into tubes for shipping. A multitude of devices for the collection of saliva are known in the art and are contemplated by the present disclosure.

Thus, in a further aspect the present disclosure provides a kit of parts which includes a saliva inducing foodstuff, such as a sweet or gum, or a sponge, and a receptacle for receiving saliva from the patient. The kit may also include a funnel which allows the patient to transfer the saliva into the receptacle easily. The receptacle may be a test tube or similar container. In preferred embodiments the receptacle may be considered to be a nasopharyngeal specimen container, or a VTM, viral transport medium. Swabs which may be suspended in the solution reagent of the disclosure are also contemplated by the present disclosure as a means for obtaining a patient sample. In yet another device to collect saliva, a sponge connected to a stick containing saliva inducing compounds may be provided separately to the collecting device. The saliva containing sponge may then be transferred to a transport tube containing a transport and release buffer. The separation is to ensure that no direct patient contact with the transport and release buffer takes place.

The methods of the disclosure comprise reacting the set of probes of the first aspect of the disclosure with a sample containing the target nucleic acid to be detected. The sample derived from the patient suspected of having an infection, such as a saliva sample, is reacted with a reagent solution. The methods of the disclosure may also be applicable to other types of biological samples. Respiratory specimens such as swabs, sputa or bronchoalveolar lavages (BALs) may be processed by the method of the present disclosure. The disclosure provides a release buffer (and incorporates the release buffer) stabilises the nucleic acid present in the sample during transportation from the patient to a testing lab. The release buffer comprises one or more polyols, one or more chaotropic agents which disrupt the structure of the infectious agent, a solvent which complexes water molecules, a reducing agent which solubilises mucus and compound which neutralises the negatively charged phosphate backbone of any nucleic acid realised from the infection agent. Table 1 provides details of preferred formulations and the range of amounts of the various components. Accordingly, the present disclosure provides a preferred formulation comprising 5 mM Tris(2-carboxyethyl) phosphine hydrochloride; 50 mM Tris/HCL pH8.3; 0.1%SDS; 95% glycerol and saturated Guanidine hydrochloride (^{~}6 M at room temperature).

**Table 1. Microbial Release Buffer ("ReBu")**

| | **Concentration** | | | | |
|---|---|---|---|---|---|
| **Compound** | **Range** | **Preferred** | **Most preferred** | Comments | **CAS number** |
| Tris(2-carboxyethyl) phosphine hydrochloride (TCEP) | 0.1 - 20 mM | 0.1 - 10 mm | 5.0 mM | Reduces S-S bonds, solubilises mucus, disrupts viral proteins, inhibits RNases | 51805-45-9 |
| Tris/HCl pH 8.3 | 10 - 100m M | 25 - 75 | 50.0 mM | Strong buffer for crude specimens; pH stabilises RNA/DNA | 77-86-1 |
| SDS | 0.001 - 0.1% | 0.001 - 0.05% | 0.001 % | Disrupts capsid | 151-21-3 |
| Triton X | 0.001 - 0.02% | 0.005 - 0.015% | 0.010 % | Disrupts capsid | |
| Glycerol | 50 - 100% | 60 -95% | 95.0 % | For the complexing of water molecules and effectively concentrating nucleic acids (e.g., RNA) | 56-81-5 |
| Guanidine hydrochloride | 3 - 8 M or 30 - 50% | 5-8 M | 6.0 (Saturated @RT) M | Guanidine hydrochloride is used in RNA isolation to dissociate nucleoproteins and inhibit RNase. Strong chaotropic agent accelerates hybridisation as negatively charged phosphate backbone is neutralised. MW = 95.53 | 50-01-1 |

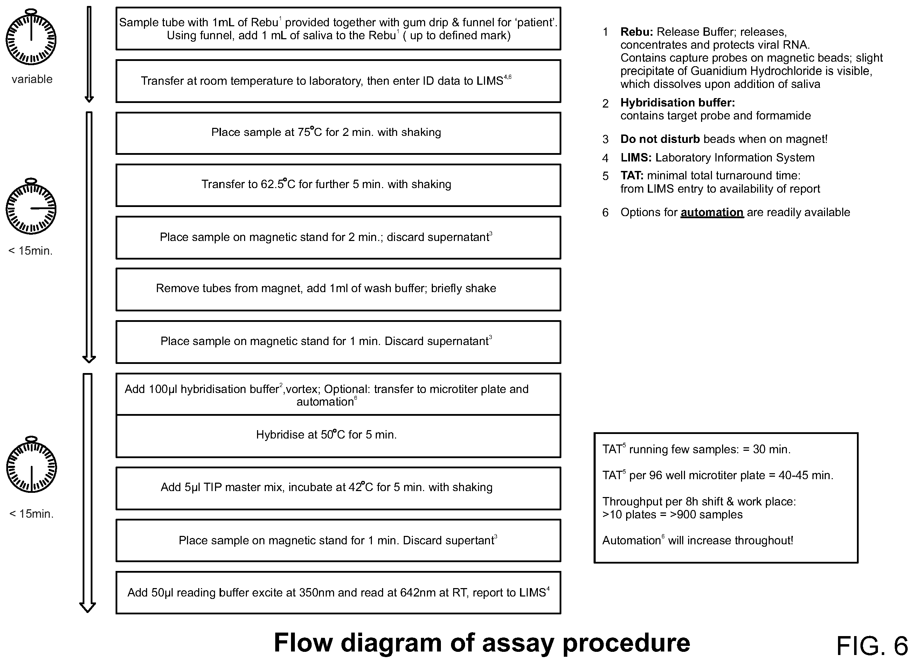

With reference to FIG. 6 the following provides a protocol for obtaining a sample from a patient suspected of having an infection.

For saliva samples the sample pack contains a plastic tube with screw cap, a funnel together with simple instructions. The contents of saliva are subject to considerable variations, especially postprandial. A gum-drop is provided as a means of inducing saliva production so as to maintain consistency in saliva composition. The components of such a gum-drop are acceptable to all ethnic backgrounds and be free of glucose. Plant based alternatives to gelatine such as agar or pectin are used. The objective is to standardise the saliva presentation as closely as reasonably possible.

The tube contains a release buffer which comprises the component parts noted in Table 1 which combines extraction, concentration, and the selective binding of viral nucleic acids. For this, the tubes already contain a nucleic acid capturing device to utilise the time between sample taking and entry to the laboratory. The device is therefore already an integral part of the assay. The nucleic acid capturing device is the release buffer which contains a nucleic acid sequence which binds to a target sequence of a nucleic acid extracted from the infectious agent. The primary design and main function is to be an easily performed sample preparation step to uncouple the timing of sample preparation and actual performance of the assay.

Blood or plasma may contain a plethora of exosomes, which may be enriched in microfluidic devices containing spirals selecting for constituents by size and presenting them in discrete outlets. An aliquot from the selected outlet containing exosomes is taken and equal volumes of release buffer containing capture beads is added. All further treatment steps follow the same procedural sequence as from saliva samples.

Aqueous release buffers that may be used in the present disclosure for stabilising nucleic acid are well known in the art. Preferably the combination of components of the release buffer of the present disclosure and used in the kits and methods of the present disclosure are designed to:
1) Liquefy highly viscous saliva or sputum samples
2) Disrupt viral capsids, bacterial, fungal or parasite cell wall
3) Complex water molecules to effectively concentrate nucleic acids
4) Inactivate RNases (and DNases)
5) Stabilise RNA
6) Provide an environment that supports and accelerates hybridisation
7) Deploy capture technology to specifically capture target sequences during transportation
8) Render samples non-infective
9) Inhibit growth of potentially contaminating micro-flora.
10) Protect from frost during transport

Chaotropic substances are well known in the art and may be used. Guanidinium thiocyanate was disregarded to avoid having a thiocyanate product in an in-vitro diagnostic device, especially as it may be in the vicinity of a patient. The chaotropic agent is therefore preferably guanidine hydrochloride (GuHCl) at a concentration of up to 6M, which is the theoretical maximum in an aqueous medium.

The polyol may also act as a solvent with equivalent molarity of GuHCl. Preferably, the molar concentration of GuHCl in glycerol is at least 3 M, preferably 4 M or 5 M, and most preferably is used at saturation concentrations of about 6 M. Alternatively, the molar concentration of glycerol in combination with >3 M GuHCl remains sufficiently high to inhibit the growth of any contaminating micro-organism as well as any nuclease activity.

GuHCl dissolved in glycerol is not only used as a chaotropic agent to disrupt, biological materials such as viral particles, but it also neutralises the negative backbone of nucleic acids. Surprisingly, it was found that GuHCl is soluble in glycerol with a molarity on par with a molarity achieved with water as solvent. In hybridisations, the negative backbones of nucleic acids reduce the hybridisation efficiency by repelling each other. The neutralised nucleic acids exert hybridisation kinetics similar to the kinetics of peptide nucleic acids due to their enhanced ability to pass cell walls. For instance, it is well known in the art that peptide nucleic acids with no negatively charged backbone have particularly favourable hybridisation characteristics, which are utilised in analogy in this disclosure to enhance the probability of the capture sequence meeting and binding the respective virus nucleic acid. The nature of buffer constituents will disrupt any viral capsid, bacterial, fungal or parasitical cell preventing growth of any potential microbial infectious agent.

This unique combination of constituents within the reagent buffer liquefies sputum, saliva and other mucous body fluids by reducing S-S bridges in protein with TCEP; the glycerol complexes water molecules, effectively increasing the concentration of any component within an aqueous "bubble"; the GuHCl functions as a chaotropic agent, releasing viral, bacterial, fungal, or parasitical nucleic acid particles and inhibiting RNases (and DNases); stabilises RNA; supports and enhances hybridisation by neutralising negatively charged phosphate back-bone of nuclei acids; concentrates nucleic acids onto a magnetic support; while rendering viruses non-infective and inhibiting the potential growth of contaminating microorganisms. The inhibitor of microbial growth is the combination of high osmotic pressure achieved by using glycerol as solvent, together with at least 3 M GuHCl after 1:1 dilution with saliva.

Many components, well known in the art, may be combined to disrupt viral particles and cells, inhibit RNases and suppress microbial growth. However, a unique set of components, as described in Table 1, was found that is able to achieve the set specifications according to both their respective physical and chemical characteristics and to work in conjunction with each other to achieve set objectives in a non-aqueous environment. In a preferred embodiment the reagent solution of the disclosure comprises glycerol in which the following are dissolved: Tris(2-carboxyethyl) phosphine hydrochloride (TCEP), Triton X, Guanidine hydrochloride (GuHCl), and Tris/HCl pH 8.3.

Table 1 provides the details of the formulation, specifying the range of possible concentrations, the preferred range, and the most preferred range. Notably, the solution is non-aqueous and no chelating agents such as EDTA, EGTA were required. It was surprising to find that a saturated solution of GuHCl could be also be achieved in glycerol, with an equivalent molarity to the molarity in water. This enables the concentration of GuHCL in glycerol after dilution with sample also to be greater than the 3 M required to maintain the chaotropic effect on protein and thus function as an RNase inhibitor and disrupt viral integrity.

In an alternative embodiment the samples may be derived from other body fluids.

The invention can detect nucleic acids from body fluids or parts thereof such as exosomes containing bacterial or viral RNA. Recent publications⁷ demonstrate the early presence of viral RNA in exosomes found in plasma, indicating that setting up an assay to detect SARS-CoV-2 RNA may cut the latent period, i.e. the period in which the respective viral RNA is not detectable using current sample preparation and detection methods. Apart from the ample availability of blood-plasma in a routine diagnostical environment, high yields plasma may be produced from finger prick blood. The use of spiral microfluidics methods are particularly suited to be combined with the teachings of this invention, where a sample will be taken from the selected outlet of the microfluidic device and treated with an equal volume of release buffer of the invention.

In one embodiment the release buffer contains a nucleic acid attached to a bead designed to capture large nucleic acids of diagnostic relevance. The capture nucleic acid (Capture Probe) binds to a nucleic acid sequence which utilises the extraction of viral nucleic acids of the infectious agent into the reagent buffer. Preferably the bead is magnetic which assists in the downstream assay. A biotinylated Capture Probe may be bound to Avidin, Neutravidin, or Streptavidin which are in turn covalently bound to the bead. Alternatively, the Capture Probe may be covalently bound to the bead via spacer arm with methods well known in the art. Accordingly in an embodiment of the disclosure the kits of the disclosure also comprise a capture probe, preferably a capture probe associated with a particle such as a magnetic bead or a capture that is biotinylated and which can bind Avidin, Neutravidin, or Streptavidin bound to a bead.

In certain methods of the disclosure once the sample is mixed with the release buffer, it is heated to a maximum of 75°C to inactivate viral particles, release the viral nucleic acid and to uncoil tertiary and secondary structures. The Capture Probe is designed to hybridise to the large infecting nucleic acid with a Tₘ between the inactivation temperature and the Tₘ of the Target Initiation probe. Upon cooling the Capture Probe will anneal and keep the large nucleic acid attached to the bead. It may be advantageous to add the Target Initiation probe before cooling to the hybridisation temperature in the cooling step to compete against a potentially preferred re-coiling of said large nucleic acid.

The Target Initiation probe is described above. The release buffer causes nucleic acids to be released from the sample. The target sequence within the released nucleic acid hybridises to a sequence within the loop of Target Initiation probe. As the extraction and initiating hybridisation reaction is able to commence immediately, no time is lost during the transportation of the sample to the lab.

In an alternative embodiment, the method described may be used for in-situ hybridisation procedures. A sample is deparaffinated according to well-known procedures, smears from swabs are made on slides, or alternatively, small (10 µl) aliquots are applied to a field on a slide and heat fixed. All further steps follow the identical procedure as described for salivary samples. The slides are immersed in ethanol for 5 minutes and dried. The Target Initiation probe is added in hybridisation buffer and incubated at 50ºC for 10 minutes. Further 5 µl Chain Loops are added and incubated at 30ºC for further 10min. The polymerisation is stopped by immersing the slide in 50% ethanol in 5 mM either Mg2+, spermidine or poly-lysine. The slides are the dried and mounting medium is applied for reading under an epi-fluorescence microscope. A further embodiment could employ an enzymatic endpoint enabling integration with existing instrumentation.

A particular aspect of the Target Initiation probe design is to choose and construct one or a plurality of probes is such a way that they are able to compete with the spontaneous re-folding of "indigenous" nucleic acid. Especially RNA viruses have long complementary sequences within highly complexed secondary and tertiary structures which are highly competitive to a successful hybridisation. This may be overcome by choosing one or a plurality of Target Initiation probes towards target sequences in very close vicinity. In addition, the employment of non-labelled helper oligonucleotides with identical thermodynamic properties to the target initiation probe is a further option to inhibit a spontaneous re-folding of viral nucleic acids, where these oligonucleotides are placed to be competitive to long helical sequences. Accordingly, it is an aspect of the invention to provide kits containing one or more helper oligonucleotides with identical thermodynamic properties to the Target Initiation probe.

In one embodiment, the present invention is used to detect the presence or absence of SARS-Cov-2. The major capsid protein of this virus is the spike protein otherwise known as the S protein or S antigen. The sequence of the S-protein is highly structured with multiple strong (GC-rich) hairpin sequences. Moreover, these tight secondary structures form a three-dimensional structure, which is virtually impenetrable for a short primer sequence structure of S-protein RNA sequence with position of target sequences).

A particular advantage of the present invention in the context of mass testing of a population for a viral infection such as COVID-19 is that the said capturing may occur upon contacting the saliva sample with the reagent mixture. Therefore, the capturing process may commence during transportation of the sample from the patient to the testing lab. There is no wasted time with respect to the optimisation of an assay's total turnaround time which is a drawback of previous mass testing methods.

The methods of the present invention allow sample sourcing to immediately flow into the extraction of nucleic acid from the bacteria or viruses or fungi in the sample and further into the detection with minimum number of steps.

Once the Target Initiation probe(s) are bound to the nucleic acid of diagnostic interest, the chain formation is initiated. The addition of the set of said Chain Loop probes to the reagent solution causes a sequential polymerisation of the Chain Loop probes and generation of a detectable signal. As a result, "a signal generating polymer" is formed. Features relating to the first aspect of the invention in relation to the Target Initiation probe set, initiating and Chain Loop probes are equally applicable to the methods of the invention.

### Probes

In one embodiment, the present disclosure provides a set of hairpin loop forming nucleic acid probes, comprising an initiating hairpin loop probe (Target Initiation probe) and one or a plurality of polymerising hairpin loop probes (Chain Loop probes) (FIG. 1). Under hybridization conditions, unbound Hairpin loops maintain their closed hairpin structure, (because the internal stem binding is thermodynamically preferred). Only in presence of a target sequence will the Target Hairpin loop bind (open) and expose its stem sequences. (The binding to the target is thermodynamically more preferred than the hairpin formation.)

Hybridisation of a Target Initiation probe to a nucleic acid target, such as DNA or RNA, causes the probe itself to present a target for further binding of Chain Loop probes in a set of Chain Loops. Binding of further Chain Loop probes to a first hybridised probe causes amplification of a signal.

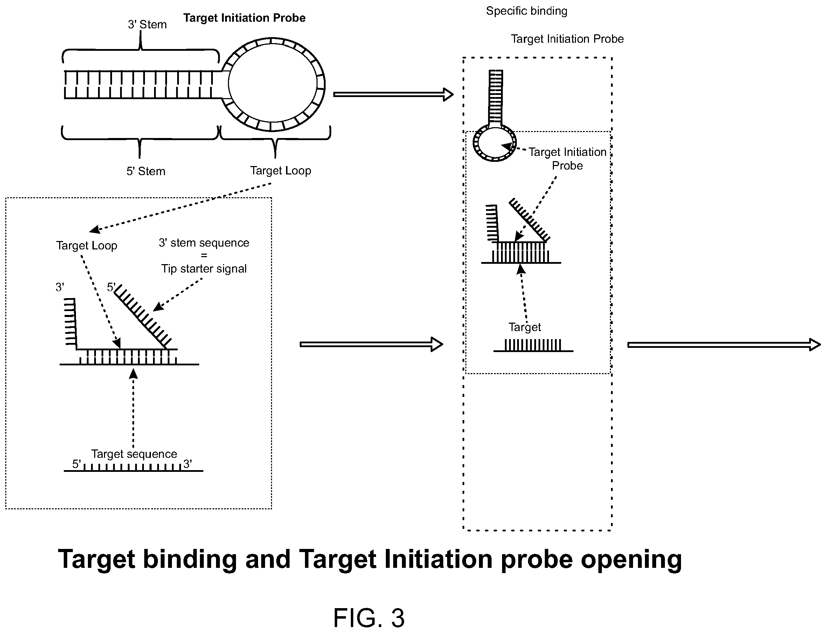

The Target Initiation probe has a region which comprises a complementary sequence to a target of choice which hybridises to the respective target nucleic acid (FIG. 3). Binding of the Target Initiation probe to the target nucleic acid causes its stem to open which then exposes a second target sequence within its stem sequence previously masked by being in the hairpin loop conformation (FIG. 3).

The present inventor designed a multiplicity of probes which act together as a set of probes to enhance the signal output from binding of the Target Initiation probe to the nucleic acid target. Each participating Chain Loop (n) in the polymerisation has a sequence within its loop structure that hybridises to a nucleic acid sequence contained within the stem structure of the previous Chain Loop probe (n-1) in the set. By designing sequences within each loop and stem it is possible to concatenate each respective probe in a sequential order to effectively form a polymer (FIG. 4a). Polymerisation happens when the Target Initiation probe is bound to the target nucleic acid. Any probes which do not bind to the target RNA or DNA remain as hairpin structures and therefore are not available to participate in the polymerisation process. Therefore, an advantage of the set of probes of the present disclosure is that non-specific hybridisation of probes is avoided and background noise can be minimised.

The newly exposed sequence that is in the stem of the first probe preferably has a complementary sequence to a sequence in the loop region of a further probe (n+1). A set of hairpin loop forming probes (Chain Loop probes) designated for polymerisation begin to sequentially form polymers only in the presence of the exposed stem sequence of said Target Initiation probe (FIG. 4a).

### In more detail:

Chain Loop I comprises a 5' stem sequence, a target loop, a 3' stem sequence and a signal generating component. Under hybridisation conditions, the target loop of Chain Loop probe I hybridises to the exposed 3' stem sequence of the open Target Initiation probe. Upon hybridisation, Chain Loop probe I adopts an open conformation, exposing its 3' and 5' stem sequences which themselves can act as a target for subsequent Chain Loop probes.

Chain Loop II comprises a 5' stem sequence, a target loop, a 3' stem sequence and a signal generating component. Under hybridisation conditions, the target loop of Chain Loop probe II hybridises to the exposed 3' stem sequence of the Chain Loop probe I. Upon hybridisation, Chain Loop probe II adopts an open conformation, exposing its 3' and 5' stem sequences which in a similar manner can act as a target for subsequent Chain Loop probes. In particular and in a preferred embodiment Chain loop II may hybridise to Chain Loop I, Chain Loop V or Chain Loop VI via its hairpin sequence, and may also be bound by the hairpin sequence of Chain loop III.

Chain Loop III comprises a 5' stem sequence, a target loop, a 3' stem sequence and a signal generating component. Under hybridisation conditions, the target loop of Chain Loop probe III hybridises to the exposed 3' stem sequence of the Chain Loop probe II. Upon hybridisation, Chain Loop probe III adopts an open conformation, exposing its 3' and 5' stem sequences which themselves can act as a target for subsequent Chain Loop probes (specifically, Chain Loop IV as currently diagrammed).

Chain Loop probe IV comprises a 5' stem sequence, a target loop, a 3' stem sequence and a signal generating component. Under hybridisation conditions, the target loop of Chain Loop probe IV hybridises to the exposed 3' stem sequence of the Chain Loop probe III. Upon hybridisation, Chain Loop probe IV adopts an open conformation, exposing its 3' and 5' stem sequences which themselves can act as a target for subsequent Chain Loop probes (specifically, Chain Loop V as currently diagrammed).

Chain Loop probe V is a Splitter probe, comprising a 5' stem sequence, a target loop sequence and a 3'stem sequence. The 5' stem sequence of Chain Loop probe V is elongated compared to Chain Loops I to IV, providing a distinguished sequence to which a further unique Chain Loop probe can hybridise. Thus, the Chain Loop probe V is able to simultaneously hybridise to three Chain Loop probes and initiate the formation of a new chain while continuing the elongation of the initial chain. In a preferred embodiment, the target loop of Chain Loop probe V hybridises to the exposed 3' stem sequence of the Chain Loop probe IV. Upon hybridisation, Chain Loop probe V adopts an open conformation, exposing its 3' and 5' stem sequences which themselves act as a target for subsequent Chain Loop probes (specifically, Chain Loop VI and Chain Loop II as currently diagrammed). Chain Loop probe V therefore starts two new cycles of chain formation (one on each exposed arm of stem sequence). Such a cycle split will repeated until all Chain Loops present are used up. The number of cycles grows exponentially with five FRET pairs formed via hybridisation per cycle, in contrast to only one beacon per cycle added in PCR.

Chain Loop probe VI comprises a 5' stem sequence, a target loop, a 3' stem sequence and a signal generating component. Under hybridisation conditions, the target loop of Chain Loop probe VI hybridises to the exposed 5' stem sequence of the Chain Loop probe V. Upon hybridisation, Chain Loop probe VI adopts an open conformation, exposing its 3' and 5' stem sequences which themselves can act as a target for subsequent Chain Loop probes (specifically, Chain Loop II as currently diagrammed).

In addition to hybridising to the exposed 3' stem sequence of the Chain Loop probe I, the target loop of Chain Loop probe II is complementary to, and therefore able to hybridise to, the 3' stem sequence of Chain Loop V and the 3' stem sequence of Chain Loop probe VI, starting a new chain of polymerisation. This permits the exponential amplification of the signal.

A set of Chain Loops are bound *via* the initial binding of Chain Loop I to a nucleotide sequence of the initiating Target initiation probe. The polymerisation continues with Chain Loop II, III, IV, V and VI. The polymerisation will continue until one of the reagents is exhausted. In certain embodiments, Chain Loops III, IV, or both III and IV may be omitted, but in a preferred embodiment, at least one of Chain Loops III and IV is included, as each additional Chain Loop amplifies the signal produced.

It can be understood by an individual skilled in the art, that in a specific embodiment the target loop of Chain Loop (n) can be designed to hybridise to the 5' stem sequence of Chain Loop (n-1), and vice versa.

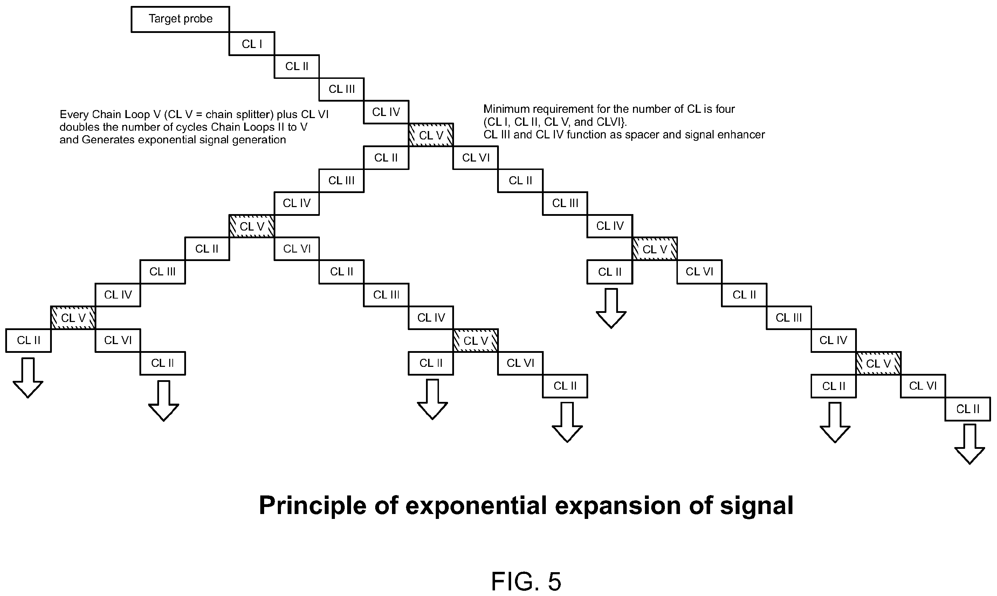

Thus, in a preferred embodiment:
a set of six Chain Loops (Chain Loop I, chain Loop II, Chain Loop III, Chain Loop IV, Chain Loop V, and Chain Loop VI) are employed, which are deemed chain segments. Each segment being one part of the polymer (FIGs. 4a-4c), in which
Chain Loop I hybridises to the 3'sequence of the Target Initiation probe's stem sequence,
the Chain Loop II hybridises to the 3' sequence of Chain Loop I, V and/or VI,
the Chain Loop III hybridises to the 3' sequence of Chain Loop II,
the Chain Loop IV hybridises to the 3' sequence of Chain Loop III,
the Chain Loop V comprising of three functional sequences; a) the loop sequence hybridising to Chain Loop IV; b) the 3' side of the stem carrying an elongated sequence to which Chain Loop VI hybridises; c) on the 5' side of the stem with a sequence complementary to the loop sequence of Chain Loop II, and to which Chain Loop II will hybridise initiating the next of a plurality of chain segments, starting a new chain of polymerisation.
the Chain Loop VI which hybridises to the 3' stem of Chain Loop V and which carries a complementary sequence to the loop sequence of Chain Loop II, which will function as a splitter starting a new chain of polymerisation (FIG. 5).

The thermodynamic properties of the Chain Loop probe are essential for their function. While initially maintaining the hairpin loop structure, under hybridisation conditions the Chain Loop probe will unfold as specific hybridisation takes place. To maintain the initial formation, the helical stem has a negative Gibbs free energy value (ΔG) under hybridisation conditions. It is well known in the art that hairpin loops have a higher specificity than respective linear oligonucleotides. In order to drive target hybridisation, the ΔG of the target loop sequence hybridising to a complementary target sequence is substantially more negative than the ΔG of helical stem formation. The higher the difference in ΔG, the faster a hybridisation will take place. Therefore, in the absence of a target nucleic acid, the Chain Loop probe adopts a closed conformation. However, in the presence of a target nucleic acid and under hybridisation conditions it is thermodynamically favourable for the Chain Loop probe to spontaneously hybridise with the target nucleic acid, adopting an open conformation.

The skilled person will be able to calculate suitable sequences using the tool found at ndbserver.rutgers.edu/ndbmodule/services/softwares.html, which enables calculation of the thermodynamic data of a given oligonucleotide sequence. The preferred database used is found following the UNAFold Web Server link, or licenses thereof.

The present disclosure provides one or a combination of detectable labels selected from labelling technologies well known in the art, some of which, but not limited to, are:
1. Radio labelled compounds together with Lanthanides forming scintillation proximity assays (SPR),
2. Proximity ligation assay (in-situ PLA),
3. Foerster (fluorescence) resonance energy transfer (FRET) with or without time resolved fluorescence (TRF),
4. When capturing is performed using capture sequences covalently bound to beads, the Chain Loops may carry a biotin moiety linked to the nucleotide with a spacer arm, and where the presence of a nucleic acid sequence is detected by washing and subsequent binding of Avidin, Streptavidin, or Neutravidin labelled with enzymes.

Each of the probes in the set of Chain Loop probes is labelled with a signal generating group which emits a detectable signal upon binding of the Target Initiation probe to a target nucleic acid followed by polymerisation of the Chain Loop probes. In some embodiments, only the Chain Loop probes contain a signal generating component.

Preferably, each Chain Loop probe carries both a donor and an acceptor fluorophore. In order to prevent intra-molecular FRET occurring, both fluorophores need to be placed with as much distance from each other as possible, as the resonance transfer decreases by 1/r². Furthermore, they need to be positioned in such a way that only upon hybridisation of Chain Loop probe (n) with Chain Loop probe (n-1) will donor and acceptor fluorophores be positioned close enough to form full FRET pairs and for an inter-molecular energy transfer to take place. In the most preferred positioning a Chain Loop probe carries one fluorophore attached to the 5'OH group of the first nucleotide. The second fluorophore is positioned in an intra-molecular position at the very beginning of the complementary 3' end of the Chain Loop that is at the 5'end of the 3'stem sequence (FIG. 4a). As presently exemplified, the internal fluorophore is attached to the base of such Chain Loop nucleotide located at the 5' end of the 3' stem sequence (thereby impacting base pairing at the position of attachment), though other sites of attachment (e.g., to the ribose backbone of a nucleic acid residue) of such internal fluorophores (subject to 1/r² proximity considerations as noted above) are also contemplated as within the scope of the instant disclosure. The positioning according to this embodiment of the instant disclosure allows the elimination of a wash step and thus having a homogeneous assay as any Chain loop probes that do not hybridise remain in the closed conformation and therefore do not generate any signal.

In an alternative embodiment, the signal generating group may be a chemical crosslinking moiety or, preferably, a Foerster (Fluorescence) Resonance Energy Transfer (FRET) pair. Preferably, the signal generating group is FRET combined with a fluorophore to form a FRET pair. The signal generating group may comprise of a FRET signal generating group (signal donor) positioned on Chain Loop probe (n) and a second fluorophore (acceptor) positioned on Chain Loop probe (n+1). The FRET group only emits a signal when the Chain Loop probes n and n+1 to which they are respectively attached are hybridised, bringing the two groups within close proximity. The emissions emanating from said chain causes the highly sensitive detection signal indicating the presence of the targeted nucleic acid sequence. However, for the detection of very low copy numbers of targets, the fluorophores typically used in the art may not generate a signal with a sufficient signal to noise ratio.

Preferably, the fluorophore in the FRET pair is one that is excited by one of the emission peaks emitted by a Lanthanide. More preferably, the first fluorophore has a chelating agent such as DOTA (dodecane tetra acetic acid), EDTA, or an analogue of such a chelating agent complexed with Europium (Eu) bound to the base of the first 5' nucleotide. The second fluorophore within the Chain Loop probe is designed to form a FRET pair in that it is excited by the Eu-emission wave length.

The positions of donor and acceptor fluorophores are expressly contemplated as freely interchangeable (subject to formation of FRET pairs upon intra-molecular hyrbridizations between respective Chain Loops). FRET pairs can be chosen from amongst the wide range of known fluorophores depending upon the filters available in an end user's fluorescent signal detection instrumentation.

Various sets of preferred probe sequences carrying fluorophore groups are listed in Table 2.

The use of FRET pairs allows the construction of a set of oligonucleotides where their respective polymerisation is target dependant and carries a unique signal generating complex with a high signal to noise ratio required to detect target sequence present at the femto- to atto- molar level from clinical specimens. Preferably a fluorophore in a FRET pair is positioned such that the emitting fluorophore is at the proximity of the 3' end of a probe sequence towards and the exciting fluorophore is positioned at the next probe in the polymer at its 5' end.

In a further embodiment, a chemical or photochemical crosslinker may be added to further improve FRET conditions and enhance the signal as well as stabilizing the entire chain by having a covalent linkage of chain elements.

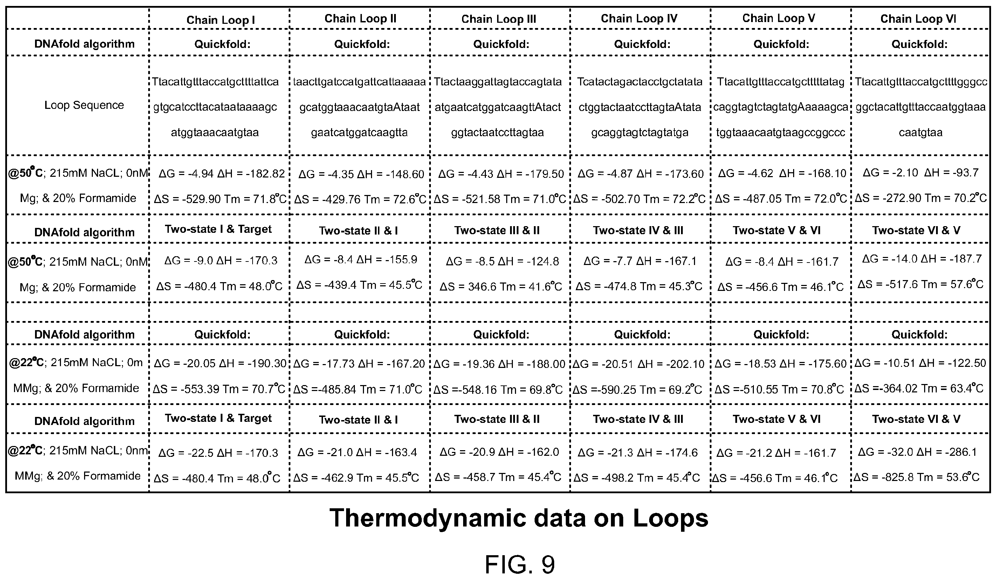

The adherence to stringent construction rules with respect to their thermodynamic properties is essential in enabling the multiplexing of oligonucleotides. Multiplexing oligonucleotides need to perform reproducibly under identical conditions while deviating substantially in their actual nucleotide sequences. The dominant factor to successfully enable multiplexing is to stringently design the hairpin loops according to their Gibbs free energy (ΔG), while allowing a limited fluctuation of the Tₘ-values. The Tₘ of each probe in the set of probes is chosen to be between 0 and 15°C higher than the assay temperature. In a preferred embodiment, the Tₘ is between 0 and 10°C above assay temperature. More preferably, Tₘ is targeted between 0 and 5°C above assay temperature. Each Chain Loop probe is designed such that its inclusion in the polymer is thermodynamically preferred over the hairpin formation under hybridisation conditions. Preferably, in the construction of hairpin loops, the ΔG of the hairpin loop is designed to be negative under hybridisation conditions at between -0.1 and - 10 kcal/mol. The most preferred values are between -1 and -5 kcal/mol. Equally, the ΔG generated when hybridising the loop sequence with the respective target needs to be controlled at +/-1.5 kcal/mol (FIG. 9).

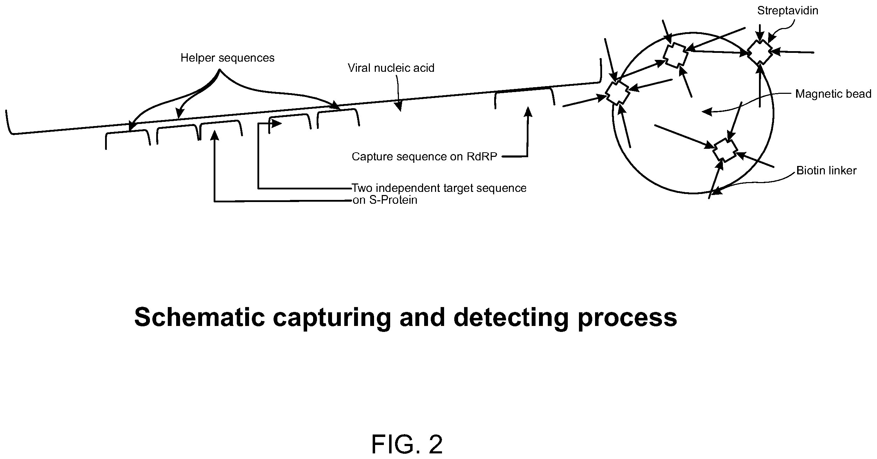

Following the principles of this invention the hybridisation events may be orchestrated to occur sequentially by designing the hybridisations to occur dependent upon the chosen temperature. Events may be determined to take place by stepwise reduction of the hybridisation temperature. With this principle in mind a plurality of assays may be formed according to respective requirements. In a preferred setup the introduced step encompasses a) the capture of a large nucleic acid, b) the hybridisation of a plurality of secondary, tertiary, and so on target specific hybridisations, and c) the initiation of the target amplification (FIG. 2).

A further aspect in capturing and the binding of target specific probes particularly in RNA is that RNA is very likely to coil into very complex two- and three-dimensional structures. The binding of oligonucleotides to highly structured target sequences may be facilitated by designing non-labelled oligonucleotides to bind juxtaposition to the labelled Target Initiation probe, helping to compete against the spontaneous re-folding of RNA (FIG. 2). When two Target Initiation probes are utilised with target binding regions close to each other they have the additional advantage of acting together to prevent the spontaneous re-folding of the RNA.

This process initiates a plurality of chains starting in analogy to the formation of dendrimers. Orchestrating Chain Loops II, V and VI the signal generation increases exponentially in a logarithmic scale at least on par to the increase in a polymerase chain reaction. The length of the polymer formed by the sequential hybridisation of the set of probes may be controlled by the concentration of each probe in the set. More preferably, blocking oligonucleotides or hairpin loops with shortened or non-cognate stem regions may be included in the hybridisation reaction which will slow or stop the polymerisation process.

Without controlling factors, the number of re-iterations is limited only by the concentration of polymerising hairpin loops in the reaction medium. It is therefore possible to detect low copy numbers of target nucleic acid by amplification of the signal.

The signal carrying nucleotides are positioned inside the hair-pin loop sequences in such a way that the two hybridising sequences position the signal carrying nucleotides in such a way that they may form FRET pairs. In a preferred embodiment the FRET pair is positioned a) at the 5' end of a loop's stem sequence and b) close to the 3' end of the stem of the loop sequence. Both will only form a FRET pair only when consecutive probes form a polymer on hybridisation. The stability of the FRET-pair will depend on strength of the hybridisation. This may be further stabilised by introducing crosslinking moieties that can be selectively triggered, using technologies well known in the art of affinity labelling. The initiation hairpin loop forming probe may also have a signal generating group for dedicated IVD diagnostic assays.

As the polymerisation is dependent upon the open availability of one defined sequence in the stem of the earlier probe in the polymer, the binding of the initiation hairpin loop forming probe to a target nucleic acid is the only trigger for the polymerisation and the formation of FRET-pairs. The loop part of the hairpin may contain any sequence of choice. This opens the application to the detection of any target sequence of diagnostic or scientific value both *in-situ* and in homogeneous liquid assays.

The initiation and polymerisation hairpin forming probes of the invention may be inexpensively constructed as non-labelled oligonucleotides. Fluorophore labels and FRET groups may be applied to the probes of the instant disclosure by methods known in the art, and are also available commercially.

The stem regions of each probe in the set need not be of the same length. The stem region not only stabilises the two- and three-dimensional structure of the hairpin probe, but also forms an active moiety in its own right, providing a further degree of freedom. In a preferred embodiment, the stem sequence is designed to be a specific target for any nucleic acid or analogue thereof. In the most preferred application either the 5' or 3' side of the stem is designed to be a target for a further hairpin loop constructed under the teachings of this disclosure. The length of this sequence is used to modulate the thermodynamic properties required to differentiate the respective ΔG and Tₘ from the binding of the primary target finding hairpin loop.

The stem sequence may either be on the 5' or 3' end of the loop sequence with equivalent preference. In an embodiment, a multiplicity of hairpin loop forming probes towards a common nucleic acid target may be positioned in very close vicinity, alternately forming chains on the 5' side and on the 3' side, to avoid steric hindrance further enhance the signal strength and sensitivity of the assay. In this embodiment, the 5' and 3' chains would mirror themselves and need to be constructed to operate under identical conditions. Their respective nucleic acid sequences in the loop part of the probe are specific for each chain. Combinations of these teachings may be used to construct a multi labelling assay, using the same principles, but enabling the detection of a multiplicity of targets in the same assay. In this case, each target loop would receive a specific set of chain forming loops, each with a specific detection system or set of FRET-pairs.

Should a higher stringency in the binding of hairpin loop forming probes be required, the instant disclosure also allows for the use of helper oligonucleotides with sequence lengths up to the length of a given loop of a hairpin probe. In a preferred application at least one helper oligonucleotides may have up to four nucleotides.

The Target Initiation probe may be produced cost efficiently as a simple unlabelled oligonucleotide, where the target sequence may be easily designed and produced for any target of commercial or scientific interest, following the principles of the instant disclosure.

This provides the option to have a cost-efficient generic detection for any target by designing the nucleic acid sequence of the loop region of the Target Initiation probe to be specific for a particular target. This offers considerable commercial advantages, as one generic detection system may be used even in a high throughput environment in the development of companion diagnostics. Accordingly, in one aspect of the instant disclosure, there is provided a kit comprising the chain loop probes.

As the polymerisation process of the present disclosure is isothermal, the polymerisation commences as a chain reaction which doubles sets of five fluorophore carrying oligonucleotides in each cycle.

### Detection

Depending on the signalling method used, appropriate readers are commercially available for detection. For FRET pairs as described above, the reading buffer outlined in Table 4b can be used.

### Examples

### Example 1 - Design of probes

In an initial probe design, two hairpin loop structured DNA-probes carrying target specific sequences of sufficient length to compete with the thermodynamic preference of binding to the RNA coding sequence of the S-protein of SARS-CoV-2 compared to refolding into a homologous structure were designed. Briefly, both probes contain two functional sequences each within a hairpin loop forming structure. The first functionality is in each loop. The loop sequences bind to virus specific sequences coding for the S-Protein with an E value of E= 9x10e-6 and 1x10e-8 (FIG. 9). The second functionality is in the stem region. One arm of the stem carries a sequence representing a target in its own right, the sequence and length of the complementary arm is chosen to provide a small negative ΔG under hybridisation conditions (50°C and 20% formamide). This is to ensure that all non-bound hairpin-loops remain in hairpin formation keeping the secondary target hidden. The negative ΔG being released when the loop sequence binds to the target will open the hairpin loop and expose the second functional sequence.

The Target Initiation probe (SEQ ID NO: 1) was reinforced by adding a second Target Initiation probe (SEQ ID NO: 2) binding to the respective complementary viral RNA to force the rigid hairpins apart (Table 2). All probes were constructed to provide equivalent free energies upon hybridisation. Moreover, the combined free energy generated upon the heterologous hybridisation was lower than the free energy generated upon homologous refolding. The proximity of all these target-directed sequences bares the danger that the probes may form neutralising duplexes. While constructing the probe sequences, in-silico controls were made to ensure that such neutralisation is thermodynamically unfavoured. The choice of target sequences was made maintaining the ΔG while avoiding the probability of these two forming competing duplexes.

The sequences of the Target Initiation probe and Chain loop probes are shown in Table 2:

**Table 2 - Sequences of Capture Probe, Target Initiation Probes and Chain Loop Probes.**

| **Target Loops (Carry no fluorophore, only signal via TIP)** | **Sequence** |
|---|---|
| SARS-CoV-2-S-Protein #1 (SEQ ID NO: 1) | att cag tgc atc ctt aca taa taa atc atc tgg taa ttt ata att ata atc agc aat tta tgt aag gat gca ctg aat |
| SARS-CoV-2-S-Protein #1 (SEQ ID NO: 2) | att cag tgc atc ctt aca taa tct gac ttc atc acc tct aat tac aaa tgt tat gta agg atg cac tga at |
| SARS-CoV-2 RdRP-capture probe (SEQ ID NO: 3) | atg gtc atg tgt ggc ggt tca cta tat gtt aaa cca ggt gga ac |
| S protein Capture sequence (SEQ ID NO: 4) | tta tgt aag gat gca ctg aat |
| | |
| **Chain Loops** | (T = Atto 390 labelled T in pos.1; A =Atto 495 labelled A in position 44) |
| Chain-I (SEQ ID NO: 5) | TtacattgtttaccatgcttttgaattcatctaggattctaattAaaagcatggtaaacaatgtaa |
| Chain-II (SEQ ID NO: 6) | TaacttgatccatgattcattaaaaagcatggtaaacaatgtaAtaatgaatcatggatcaagtta |
| Chain-III (SEQ ID NO: 7) | TtactaaggattagtaccagtataatgaatcatggatcaagttAtactggtactaatccttagtaa |
| Chain-IV (SEQ ID NO: 8) | TcatactagactacctgctatatactggtactaatccttagtaAtatagcaggtagtctagtatga |
| Chain-V (SEQ ID NO: 9) | TtacattgtttaccatgctttttatagcaggtagtctagtatgAaaaagcatggtaaacaatgtaAgccggccc |
| Chain Loop VI (SEQ ID NO: 10) | TtacattgtttaccatgcttttgggccggcttacattgtttaccAatggtaaacaatgtaa |
| Chain Initiator (SEQ ID NO: 11) | biotin-attatgtaaggatgeaetgaat |

For the sequences of Table 2 above, Atto390 is used to label T at position 1, and Atto495 is used to label A at position 44 of Chain Loop probes I to VI.

After completion of this step the plate is transferred to 37°C and the polymerising oligonucleotides are added for signal amplification. The reading of the plates is made with micro-titre plate reader readily available on the market.

**Table 3 - Additional Sequences of Capture Probe, Target Initiation Probes and Chain Loop Probes.**

| **Target Loops (Carry no fluorophore, only signal via TIP)** | **Sequence** |
|---|---|
| SARS-CoV-2-S-Protein #1 (SEQ ID NO: 12) | attcagtgcatccttacataatgactgaagtagtggagattaatgtttacttatgtaaggatgcactgaat |
| SARS-CoV-2-S-Protein #1 (SEQ ID NO: 13) | attcagtgcatccttacataattttagtagaccattaaatattaatattagtcgttattatgtaaggatgcactgaat |
| SARS-CoV-2 Capture Probe added to NHS activated magnetic Sepharose beads - carries no fluorophore (SEQ ID NO: 14) | AMINOhexyl-Spacer 9(TEG)-atgtgagattaaagttaactacatctac |
| | |
| **Chain Loops - Example of TIP Set of Six Chain Loops** | (X = FRET Donor (Atto 390) labelled at pos.1; Capital A or T at pos. 44 indicates Atto 495 labelled on the base) |
| Chain-I (SEQ ID NO: 15) | XttacacactcagacctagctaaattcagtgcatccttacataaTttagctaggt |
| Chain-II (SEQ ID NO: 16) | XtaacttgatccatgattcattattagctaggtctgagtgtgtaAtaatgaatcatgg |
| Chain-III (SEQ ID NO: 17) | XttactaaggattagtaccagtataatgaatcatggatcaagttAtactggtactaatc |
| Chain-IV (SEQ ID NO: 18) | XtcatcctagcctaccggctatatactggtactaatccttagtaAtatagccggt |
| Chain-V (SEQ ID NO: 19) | XttacacactcagacctagctaatatagccggtaggctaggatgAttagctaggtctgagtgtgtaa |
| Chain Loop VI (SEQ ID NO: 20) | XttacacactcagacctagctaattacacactcagacctagctaAttagctaggt |
| | |
| **Chain Loops-Exemplified Set of Five Chain Loops Used for Successful SARS-CoV-2 Detection** | (X = FRET Donor (Atto 390) labelled at pos.1; Capital A, G or C at internal position 36 (I), 37 (II), 38 (III, V) or 43 (VI) indicates Atto 495 labelled on the base) |
| Chain-I (SEQ ID NO: 21) | XaccactgtctacctaccttcagtgcatccttacatAggtaggtagacagtggt |
| Chain-II (SEQ ID NO: 22) | XtttcttgatccatgattctttagggtaggtagacaGattagaatcatggatcaagaaa |
| Chain-III (SEQ ID NO: 23) | XaagttcataactatggatcaagaatcatggatcaAtgatccatagttatgaactt |
| Chain-IV | omitted |
| Chain-V (SEQ ID NO: 24) | XaccactgtctacctaccgatccatagttatgaaCggtaggtagacagtggtattagtggc |
| Chain Loop VI (SEQ ID NO: 25) | XaccactgtctacctaccagaagccactaataccactgtctacCagagggtaggtagacagtggta |

As shown in FIG. 4, a first Chain Loop probe binds to the exposed unique stem sequence presented by the Target Initiation probe, and opens a second unique sequence for the following second Chain Loop probe. The second Chain Loop forming probe exposes a unique sequence in its loop for a third Chain Loop probe to hybridise at its stem. Upon binding the third Chain Loop probe in the polymer a unique sequence is exposed in the loop of the third probe, and so on through to the fifth Chain Loop probe.

In this example, the 5' stem of the fifth Chain Loop carries the same sequence as the first Chain Loop probe. The second Chain Loop probe will therefore bind to both the first Chain Loop probe and the fifth Chain Loop probe. In order to have the initiation and polymerisation probes to perform sequentially, their respective thermodynamic and assay characteristics need to be fine-tuned. This polymerisation, however, is linear.

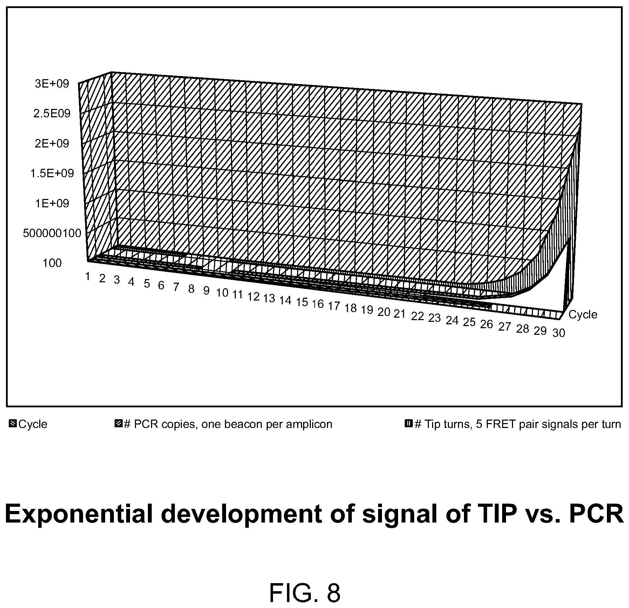

In order to amplify the signal exponentially, a modification is introduced to Chain Loop five and a sixth Chain Loop probe is introduced. The 3' end of Chain Loop 5 is elongated to build a further unique target sequence to which Chain Loop probe six carries a complementary sequence in its loop. The stem of Chain Loop probe six carries identical sequences to Chain Loop probe one and will start a new independent line of polymerisation (FIG. 5). In this exponential series each internal cycle carries 5x the signal of a single FRET pair, resulting in an exponential growth in signal strength (FIG. 8).

The respective thermodynamic data was calculated using the DNAfold algorithms (unafold.rna.albany.edu/?q=DINAMelt) and the results are summarized in FIG. 9. Generally, Termination of polymerisation may be induced by introducing a terminating "nonsense" sequence. The subsequent reduction of the assay temperature will close all non- bound polymerisation hairpin loop forming probes to form hairpin loops, and the fluorophores of the free hairpin loops will not be in sufficient proximity to form FRET-pairs. The sequential unrolling of events is demonstrated in FIG. 8, where the individual steps of chain formation is demonstrated.

### Example 2 - Kinetics of Polymerisation

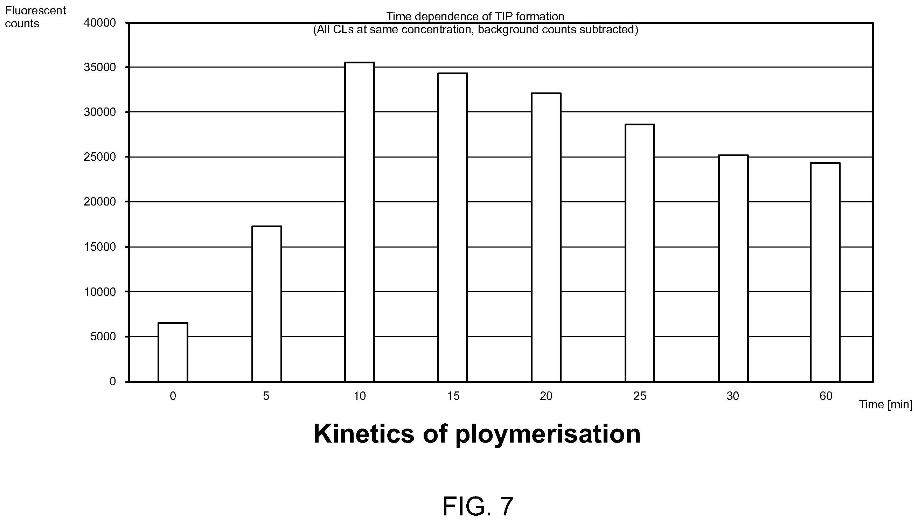

The 3' stem sequence of Target Initiation probe (SEQ ID NO: 11) was fixed to a well. 100 µl of hybridisation buffer and 5 µl of TIP mastermix containing 10pM of Chain Loops I to VI (Table 4a) was added, and the mixture incubate at 42 ºC for 5 minutes, with shaking. Sample was placed on a magnetic stand for 1 minute, and the supernatant discarded, 50 µl of reading buffer (Table 4b) was added, and the sample excited at 350 nm and read at 642 nm on a Perkin Elmer Victor 3V, 1420 Multilabel Counter. The kinetics of polymerisation is shown in FIG. 7.

**Table 4a - Hybridisation Buffer Constituents for 10.00 ml**

| **Hybridisation buffer** | **Stock conc.** | **Volume from stock** | **End concentration** |
|---|---|---|---|
| Temperature | | | 50(°C) |
| mM NaCl | 5000 mM | 0.43 ml | 215 |
| mM MgCl₂ | 100 mM | 1.50 ml | 15 |
| % SDS | 10% | 0.0001 ml | 0.01% |
| % Formamide | 99.5% | 2.00 ml | 20% |
| EDTA | 500 mM | 0.00 ml | 0.00mM |
| Tris/HCl pH | 1000 mM | 0.20 ml | 20mM |
| pM probe | pg | | 5pg |
| H₂O | | 5.87 | |

**Table 4b - Reading Buffer Constituents for 10.00 ml**

| **Reading Buffer** | **Stock conc.** | **Volume from stock** | **End concentration** |
|---|---|---|---|
| Glycerol | | 7.57 ml | |
| mM NaCl | 5000 | 0.43 ml | 215 mM |
| mM MgCl₂ | 100 | 1.50 ml | 15 mM |
| Tris/HCl pH8.3 | 1000 | 0.50 ml | 50 mM |

### Example 3 - Rapid, robust and quantitative TIP-mediated detection of SARS-CoV-2 target nucleic acid sequences in solution

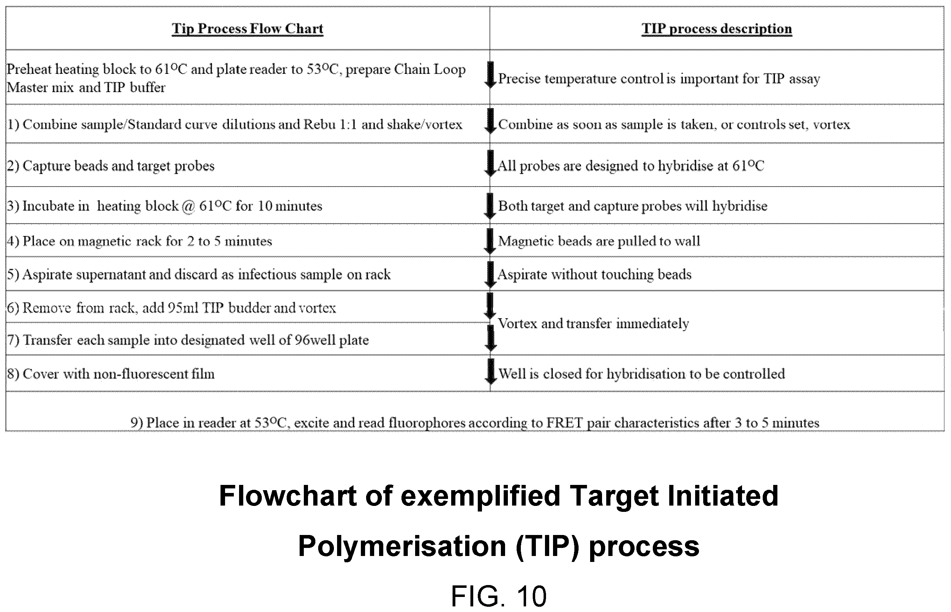

TIP-mediated detection of a SARS-CoV-2 target nucleic acid was first attempted in solution, using the SARS-CoV-2 S-Protein-encoding RNA Capture probe, Target Initiation probes and Chain Loop probes (SEQ ID NOs: 21-25, set of five) disclosed in Table 3. The TIP process of FIG. 10 was performed with the nucleic acid probes of Table 3, using the TIP Chain Loop Master mix of Table 5a and the TIP assay buffer of Table 5b, and capture beads (probe) attached to magnetic Sepharose beads at a final concentration of 1 fmol/µl.

**Table 5a - TIP Chain Loop Master mix**

| | **Final Concentration** |
|---|---|
| Chain loop I | 1 fmol/µl |
| Chain loop II | 1 pmol/µl |
| Chain loop IV | 1 pmol/µl |
| Chain loop V | 1 pmol/µl |
| Chain loop VI | 1 pmol/µl |

**Table 5b - TIP assay buffer**

| | **For 200 ml** | **Final concentration** |
|---|---|---|
| Dextran | 25g | 248 mM |
| Formamide (freshly deionised) | 40ml | |
| 5M NaCl | 8.6ml | 214.9978 mM |
| 1M Tris HCl | 4ml | 20 mM |
| 5mM MgCl | 1ml | 25uM |
| 10% SDS | 200ul | |
| 100mM Spermidine | 10ml | 5mM |
| Buffer water | top up to 200ml | |

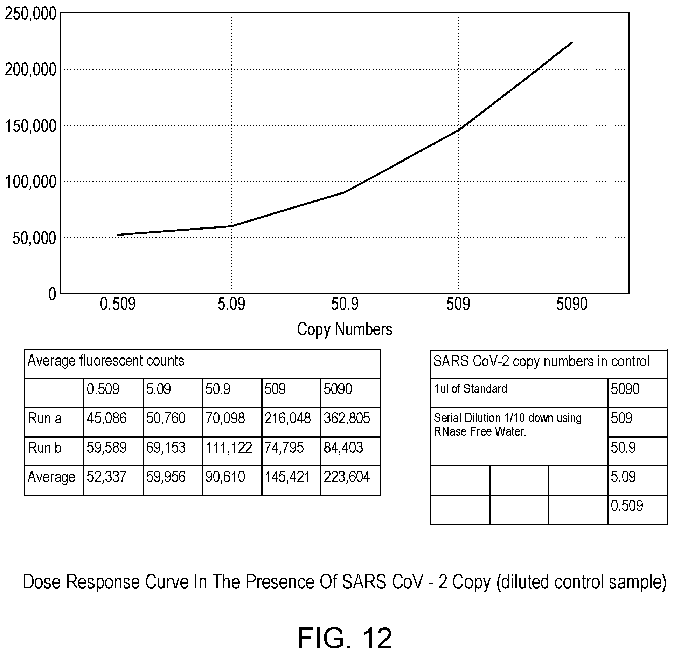

TIP-mediated signal rapidly amplified (FIG. 11) and successfully detected the presence of SARS-CoV-2 viral RNA comprising a target sequence recognized by the Target Initiation probe of the TIP process, when the viral target RNA was either directly formulated in microbial release buffer ("ReBu" of Table 1, which is a sputum liquefier, RNA extraction and transport medium) or was first formulated in water and then mixed with an equal proportion of ReBu ("50% ReBu", which was employed as reflecting the final assay of Example 4 below, where a volume of a test sample, e.g., waste water or saliva, can be added to 1 mL of ReBu in a 2 mL screw-cap vial, and can then be assayed). Both viral target RNA-containing samples ("ReBu" and "50% ReBu") exhibited rapid signal production at levels over the water only (no target RNA) control. Repeated plate reading cycles of approximately 65 seconds in length were performed to ensure that signal reached a plateau value, and interestingly, the greatest nucleic acid detection signal levels over background occurred in the approximately 2-3 minute timeframe after TIP initiation, after which a gradual decay in RNA-detecting signal levels over control (no nucleic acid and/or background levels) occurred. Thus, rapid detection of a SARS-CoV-2 target sequence in solution was achieved.To examine whether TIP-mediated detection of SARS-CoV-2 was quantitative, the TIP process was performed upon a SARS-CoV-2 target RNA serial dilution series in RNase free water (SARS-CoV-2 copy numbers of 5090, 509, 50.9, 5.09 and 0.509, respectively). Application of the TIP process (again using the SARS-CoV-2 S-Protein-encoding RNA Capture probe, Target Initiation probes and Chain Loop probes (SEQ ID NOs: 21-25, set of five) disclosed in Table 3) to the SARS-CoV-2 target RNA serial dilution series yielded a robust dose response curve (FIG. 12).

The exemplified TIP process was therefore identified to be robust, rapid and quantitative for specific detection of a target RNA sequence in solution.

### Example 4 - Protocol for detecting presence of SARS-COv-2 infection in a patient sample:

### Treatment of samples:

With reference to FIG. 8, the following provides a protocol for obtaining a sample from a patient suspected of having an infection.

For saliva samples the sample pack contains a plastic tube with screw cap, a funnel together with simple instructions. The contents of saliva are subject to considerable variations, especially postprandial. A gum-drop is provided as a means of inducing saliva production so as to maintain consistency in saliva composition. The components of such a gum-drop are acceptable to all ethnic backgrounds and be free of glucose. Plant based alternatives to gelatine such as agar or pectin are used. The objective is to standardise the saliva presentation as closely as reasonably possible.

The tube contains a release buffer which comprises the component parts noted in Table 1 as most preferred, and combines extraction, concentration, and the selective binding of viral nucleic acids. For this, the tubes already contain a Capture Probe SEQ ID NO: 3 device to utilise the time between sample taking and entry to the laboratory. The device is therefore already an integral part of the assay. The nucleic acid capturing device is the reagent buffer which contains a nucleic acid sequence which binds to a target sequence of a nucleic acid extracted from the infectious agent. The primary design and main function is to be an easily performed sample preparation step to uncouple the timing of sample preparation and actual performance of the assay.

For plasma samples enriched for exosomes, sample of the selected outlet fluid are taken and equal volumes of release buffer containing capture beads. All further treatment steps follow the same procedural sequence as from saliva samples.

### Detection of SARS-CoV-1 & SARS-Cov-2 RNA

In order to capture viral nucleic acids, biotinylated oligonucleotides with sequences complementary to SARS-CoV RNA-dependent-RNA polymerase (RdRP) bound to Neutravidin or Streptavidin coated magnetic beads are used which capture both SARS-CoV-1 (20003) and SARS-CoV-2 RNA. Surprisingly, a sequence was found (SEQ ID NO: 3) which is present in all SARS-CoV genomic sequences submitted to the NCBI data base, with a length to provide sufficient thermodynamic force to selectively extract SARS-CoV RNA directly from the sample/buffer mix at hybridisation temperature specific for the respective sequence. The BLAST analysis in the NCBI data base gives an E value of E=3x10e-4. The hybridisation temperature may range between room temperature (RT =20°C) and 95°C. The preferred hybridisation temperature is between 37°C and 80°C. The most preferred hybridisation temperature for the Target Initiation probe is 62.5°C. The temperatures may be achieved by direct heat or a combination of heat and Formamide as is well known in the art.

In the laboratory, the sample is recorded (LIMS) and the tube is vortexed and incubated at 75°C for 2 minutes and for further 5 minutes at 62.5°C to discriminate any unspecific hybridisation of viral nucleic acids. The tube is then placed on a magnetic separator for a minute and the supernatant is discarded. On and off the magnet, the beads are briefly washed with hybridisation buffer. The beads in fresh buffer is then transferred in duplicate 100 µl amounts to a microtiter-plate and hybridisation buffer (Table 4a). The Target Initiation probe are added and incubated at a temperature equivalent to 62.5°C. Only Target Initiation probes bind to a target sequence and then expose unique stem sequences, otherwise masked in the helical stem of the hairpin loop. This specific sequence initiates a cascade of signal generating events. 100 µl of hybridisation buffer will be added. The plate is placed on a magnet to draw the beads out of solution. The hybridisation buffer is discarded and reading buffer (Table 4b) is added. The sequences contained within the loop of the Target Initiation probe are complementary to parts of the virus's S-protein sequences and specifically hybridise at a temperature equivalent to 62.5°C. The hybridisation is evaluated in a suitable reader at room temperature.

In detail, the polymerising cascade is initiated by adding the set of Chain Loop probes recited in Table 2. A first Chain Loop probe (Chain Loop probe I, SEQ ID NO: 5) binds to the exposed unique stem sequence presented by the initiating Target Initiation probe hybridised to the target, and opens a 2^{nd} unique sequence for the following Chain Loop probe to bind. The 2^{nd} Chain Loop probe (Chain Loop probe II SEQ ID NO: 6) binds exposing a unique sequence for a 3^{rd} Chain Loop probe (Chain Loop probe III SEQ ID NO: 7). Upon binding the 3^{rd} Chain Loop probe a unique sequence for the 4^{th} Chain Loop (Chain Loop probe IV, SEQ ID NO: 8 ) is exposed. The same process occurs with the 5^{th} Chain Loop (Chain Loop V SEQ ID NO: 9). Upon binding the 5^{th} Chain Loop probe a unique sequence for the 6^{th} Chain Loop probe (Chain Loop Probe VI, SEQ ID NO: 10) is exposed. The 6^{th} Chain Loop probe binds, exposing a unique sequence for the 2^{nd} Chain Loop probe (Chain Loop probe II, SEQ ID NO: 6). Additionally, the 3'stem of the 5^{th} Chain Loop probe carries the same sequence as the 1^{st} Chain Loop probe. The 2^{nd} Chain Loop probe will therefore bind to the 1^{st} 5^{th} and 6^{th} Chain Loop probe. The binding to the 5^{th} Chain Loop probe therefore triggers a full new cycle.

Atto390 is used to label T at position 1, and Atto495 is used to label A at position 44 of Chain Loop probes I to VI. The positioning of the FRET groups is such that only bound probes will be positioned with sufficient proximity to form the full signal of a FRET pairs. In a further embodiment, the emission signal generating portion of the FRET pair is DOTA-Europium and may be positioned preferably at the 5' end of the stem of the Chain Loop probe such that the FRET pair are positioned in sufficient proximity to generate a signal. This opens the application of time resolved fluorescence applications to generate a high signal to noise ratio.

Upon the binding of the initiation probe to the target nucleic acid causes the polymerising hairpin loop forming probes to polymerise and an amplified signal is generated. The signal is detected using standard fluorescent techniques.

The present disclosure therefore provides methods for the *in-situ* detection of an infectious agent in a sample without extraction in less than one hour.

### Citations:

1. Dirks RM, Pierce NA (2004) Triggered amplificationby hybridization chain reaction. Proc. Natl Acad Sci U S A 101(43):15275-15278
2. Srinivas N, Ouldridge TE, Sulc P, Schaeffer JM, Yurke B, Louis AA, Doye JPK, Winfree E; (2013) On the biophysics and kinetics of toehold-mediated DNA strand displacement.
3. Zeng et al. Nonlinear hybridization chain reaction-based functional DNA nanostructure assembly for biosensing, bioimaging applications; Biosensors and Bioelectronics, Volume 173, 1 February 2021, 112814; doi.org/10.1016/j.bios.2020.112814
4. Yan Shan A., and Lin-You LY (2016) Rational design of hybridization chain reaction monomers for robust signal amplification. Chem Cummun. 54, 4219-4222.
5. Kwei et al. (2020) In situ genetically cascaded amplification for imaging RNA subcellular locations. J. Am. chem. Soc. 142, 2968-2974.

All patents and publications mentioned in the specification are indicative of the levels of skill of those skilled in the art to which the disclosure pertains.

One skilled in the art would readily appreciate that the present disclosure is well adapted to carry out the objects and obtain the ends and advantages mentioned, as well as those inherent therein. The methods and compositions described herein as presently representative of preferred embodiments are exemplary and are not intended as limitations on the scope of the disclosure. Changes therein and other uses will occur to those skilled in the art.

In addition, where features or aspects of the disclosure are described in terms of Markush groups or other grouping of alternatives, those skilled in the art will recognize that the disclosure is also thereby described in terms of any individual member or subgroup of members of the Markush group or other group.

The use of the terms "a" and "an" and "the" and similar referents in the context of describing the disclosure (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein.

All methods described herein can be performed in any suitable order unless otherwise indicated herein or otherwise clearly contradicted by context. The use of any and all examples, or exemplary language (e.g., "such as") provided herein, is intended merely to better illuminate the disclosure and does not pose a limitation on the scope of the disclosure unless otherwise claimed. No language in the specification should be construed as indicating any non-claimed element as essential to the practice of the disclosure.

Embodiments of this disclosure are described herein, including the best mode known to the inventors for carrying out the disclosed invention. Variations of those embodiments may become apparent to those of ordinary skill in the art upon reading the foregoing description.

The disclosure illustratively described herein suitably can be practiced in the absence of any element or elements, limitation or limitations that are not specifically disclosed herein. Thus, for example, in each instance herein any of the terms "comprising", "consisting essentially of", and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present disclosure provides preferred embodiments, optional features, modification and variation of the concepts herein disclosed may be resorted to by those skilled in the art, and that such modifications and variations are considered to be within the scope of this disclosure as defined by the description and the appended claims.

It will be readily apparent to one skilled in the art that varying substitutions and modifications can be made to the invention disclosed herein without departing from the scope of the invention.. The present disclosure teaches one skilled in the art to test various combinations and/or substitutions of chemical modifications described herein toward generating conjugates possessing improved contrast, diagnostic and/or imaging activity. Therefore, the specific embodiments described herein are not limiting and one skilled in the art can readily appreciate that specific combinations of the modifications described herein can be tested without undue experimentation toward identifying conjugates possessing improved contrast, diagnostic and/or imaging activity.

The inventors expect skilled artisans to employ such variations as appropriate, and the inventors intend for the disclosure to be practiced otherwise than as specifically described herein.

## Claims

1. A kit for the detection of a target nucleic acid of interest the kit comprising:
a) A hairpin Target Initiation probe and
b) A set of multiple hairpin Chain Loop probes to amplify a detectable signal comprising a signal generating component contained within the Chain Loop probe;
**characterised in that**:
the Target Initiation probe in its closed configuration is a hairpin nucleic acid comprising complementary 3' and 5' stem sequences to form a double stranded stem portion and a single stranded target loop portion comprising a sequence complementary to the target nucleic acid;
further **characterised in that** a first Chain Loop probe comprises a hairpin loop structure with a sequence in the loop complementary to the 5' or 3' stem sequence of the Target Initiation probe and a signal generating component;
further **characterised in that** a second Chain Loop probe comprises a hairpin loop structure with a sequence in the loop complementary to the 5' or 3' stem sequence of the first Chain Loop probe and a signal generating component;
further characterised that subsequent Chain Loop probes comprise a hairpin loop structure with a sequence complementary to the 5' or 3' stem sequence of a prior Chain loop probe and a signal generating component;
wherein on binding of the target nucleic acid to the Target Initiation probe the stem region of the Target Initiation probe opens and permits hybridisation to the loop region of the first Chain Loop probe and causes sequential polymerisation of multiple Chain Loop probes to form a polymer capable of generating a signal; and wherein one of the Chain Loop probes comprises a hairpin loop structure with a further nucleic acid sequence extending from the 5' or 3' stem sequence which hybridises to the target loop of another Chain Loop probe splitting the chain formation into two chains to form a dendrimer capable of generating a signal.

2. A kit as claimed in claim 1, wherein the signal generating component of the Chain Loop probes comprises one or more groups capable of generating a signal through fluorescence or chemiluminescence

3. A kit as claimed in any preceding claim, wherein the signal generating component of the Chain Loop probes comprises a Förster (Fluorescence) Resonance Energy Transfer (FRET) reporter group pair, such that in the closed position the acceptor fluorophore and donor fluorophore are separated to prevent resonance transfer and generation of a detectable signal.

4. A kit as claimed in any preceding claim, wherein the final Chain Loop probe comprises a nucleic acid sequence in its stem that is complementary to a sequence of a preceding Chain Loop probe.

5. A kit as claimed in any preceding claim, wherein the Gibbs free energy of binding of the Target Initiation probe and of one or more Chain Loop probes is negative under respective hybridisation conditions and the Tₘ of the stem formation is higher than the effective hybridising temperature of the Chain Loop probes.

6. A kit of any preceding claim, wherein the free energy (ΔG) generated upon respective hybridisation of any set of Chain Loop probe is within a range of ΔG = -X +/- 1.5kcal/mol, where X equals - 21kcal/mol at 50°C plus 20% Formamide and 215mM NaCl.

7. A kit of any preceding claim, wherein Chain Loop probe carries a target loop with a unique sequence.

8. A kit of any preceding claim, wherein the 5' or the 3' stem forming sequence of the Target Initiation probe and Chain Loop probe thermodynamically favours hybridisation with a complementary sequence over the base-pairing of its respective hairpin loop, and wherein the Gibbs free energy of stem formation of each probe is less negative than the Gibbs free energy of hybridisation with a target nucleic acid sequence.

9. A kit as claimed in claims 3 to 7, wherein the reporter pair generates a detectable signal only when a Chain Loop probe (n) binds to a Chain Loop probe (n-1) and the hybrid brings the donor and acceptor fluorophores close enough to enable Förster (Fluorescence) Resonance Energy Transfer (FRET) or for a pair or a chemical crosslinking groups to form a chemical cross-link.

10. A kit as claimed in claims 2 to 9, wherein the Chain Loop probe comprises a reporter group bound at the 3' end of the sequence within its loop and at the 5' end of the stem sequence, or *vice versa,* and wherein the reporter group generates a detectable signal only when a Chain Loop probe (n) binds to a Chain Loop probe (n-1) and the hybrid brings the donor and acceptor fluorophores close enough to enable Förster (Fluorescence) Resonance Energy Transfer (FRET).

11. A kit of any preceding claim wherein the reporter FRET pair is:
Europium and a fluorophores capable of being excited by the emission of fluorescent light emitted from Europium, preferably wherein the Europium is complexed with a chelating agent, optionally wherein the chelating agent is DOTA, EDTA or a DOTA or EDTA analogue, and the fluorophore is excited by the Europium emission peak at 620nm;
Terbium and a fluorophore capable of being excited by the emission of fluorescent light emitted from Terbium, preferably wherein the Terbium is complexed with a chelating agent, optionally wherein the chelating agent is DOTA, EDTA or a DOTA or EDTA analogue, and the fluorophore is excited by the Terbium emission peak at 495nm; or
Samarium and a fluorophore capable of being excited by the emission of fluorescent light emitted from Samarium, preferably wherein the Samarium is complexed with a chelating agent, optionally wherein the chelating agent is DOTA, EDTA or a DOTA or EDTA analogue, and the fluorophore is excited by the Samarium emission peak at 350nm.

12. A kit as claimed in claim 1, wherein Chain Loop probes carry biotin moieties as binding points for biotin/streptavidin-based detection systems.

13. A kit as claimed in any preceding claim comprising two or more set of Target Initiation probes and two or more sets of Chain Loop probes capable of reporting on the presence or absence of two or more target nucleic acids.

14. A kit as claimed in any preceding claim further comprising a Capture probe; optionally wherein the Capture probe is linked to a particle, further optionally wherein the particle is a magnetic particle.

15. A composition comprising a set of probes with sequences selected from the group consisting of: SEQ ID NOs: 21-25; SEQ ID NOs: 5-10; and SEQ ID NOs: 15-20.

16. A method for selecting sequences for a set of four or more hairpin loop nucleic acid probes capable of hybridizing linearly or exponentially with one another in the presence of a target sequence to generate a kit according to claim 1, the method comprising:
identifying candidate sequences for a first hairpin loop probe, a second hairpin loop probe, a third hairpin loop probe, a fourth hairpin loop probe, and optionally successive additional hairpin loop probes, wherein each of the first hairpin loop probe, the second hairpin loop probe, the third hairpin loop probe, the fourth hairpin loop probe, and the optionally successive additional hairpin loop probes:
(i) possesses a hairpin structure, wherein the probe comprises 5'-terminal and 3'-terminal complementary, annealed stem loop sequence regions, optionally comprising a terminal overhang between 5' and 3' termini, and a single-stranded hairpin loop sequence that joins the annealed stem sequence regions in a solution lacking other nucleic acid sequences, wherein the annealed stem sequences of each hairpin loop are selected to possess a higher Gibbs free energy (ΔG) value in solution for intra-molecule stem loop structure formation than the ΔG value for inter-molecule annealing between the single-stranded hairpin loop sequence and its target sequence in a distinct oligonucleotide (i.e., the distinct oligonucleotide being an initiator target sequence or a complementary sequence within a stem loop of a distinct hairpin probe); and
(ii) possesses a 5'- or 3'-terminal first fluorophore modification and a second fluorophore modification at an internal nucleotide residue position adjacent to or within the single-stranded hairpin loop sequence;
wherein thermodynamic properties including Gibbs free energy (ΔG) values of the candidate sequences are predicted or evaluated to identify a set of four or more hairpin loop nucleic acid probe sequences wherein:
the single-stranded hairpin loop sequence of the first hairpin loop probe is complementary to a target sequence not found within the set of hairpin loop probe sequences, wherein the single-stranded hairpin loop sequence of the first hairpin loop probe is capable of annealing to the target sequence with a ΔG value that is lower than the ΔG value for intra-molecule first hairpin loop probe stem loop structure formation;
the single-stranded hairpin loop sequence of the second hairpin loop probe is complementary to at least one stem region sequence of the first hairpin loop probe and the single-stranded hairpin loop sequence of the second hairpin loop probe is capable of annealing to the complementary stem region sequence of the first hairpin loop probe with a ΔG value that is lower than the ΔG value for intra-molecule second hairpin loop probe stem loop structure formation;
the single-stranded hairpin loop sequence of the third hairpin loop probe is complementary to at least one stem region sequence of the second hairpin loop probe and the single-stranded hairpin loop sequence of the third hairpin loop probe is capable of annealing to the complementary stem region sequence of the second hairpin loop probe with a ΔG value that is lower than the ΔG value for intra-molecule third hairpin loop probe stem loop structure formation; and
the single-stranded hairpin loop sequence of the fourth hairpin loop probe is complementary to at least one stem region sequence of the third hairpin loop probe and the single-stranded hairpin loop sequence of the fourth hairpin loop probe is capable of annealing to the complementary stem region sequence of the third hairpin loop probe with a ΔG value that is lower than the ΔG value for intra-molecule fourth hairpin loop probe stem loop structure formation,
thereby selecting sequences for a set of four or more hairpin loop nucleic acid probes capable of hybridizing linearly or exponentially with one another in the presence of a target sequence.

17. The method of claim 16, wherein each of the first hairpin loop probe, the second hairpin loop probe, the third hairpin loop probe, the fourth hairpin loop probe, and the optionally successive additional hairpin loop probes possesses a total length of 120 nucleotides or less, optionally 100 nucleotides or less, optionally 90 nucleotides or less, optionally 80 nucleotides or less, and optionally 70 nucleotides or less.

18. The method of claim 16 or 17, wherein:
the hairpin sequence of the first hairpin loop probe is capable of annealing to the target sequence with a ΔG of at least about -10 kcal/mol ± 1.5kcal/mol;
the hairpin sequence of the second hairpin loop probe is capable of annealing to the complementary stem region sequence of the first hairpin loop probe with a ΔG of at least about -10 kcal/mol ± 1.5kcal/mol;
the hairpin sequence of the third hairpin loop probe is capable of annealing to the complementary stem region sequence of the second hairpin loop probe with a ΔG of at least about -10 kcal/mol ± 1.5kcal/mol;
the hairpin sequence of the fourth hairpin loop probe is capable of annealing to the complementary stem region sequence of the third hairpin loop probe with a ΔG of at least about -10 kcal/mol ± 1.5kcal/mol; and/or
the hairpin sequence of the (n+1)^{th} hairpin loop probe is capable of annealing to the complementary stem region sequence of the n^{th} hairpin loop probe with a ΔG of at least about -10 kcal/mol ± 1.5kcal/mol.

19. The method of any one of claims 16 to 18, further comprising synthesizing the set of four or more hairpin loop nucleic acid probes, thereby selecting and preparing sequences for the set.

20. The method of any one of claims 16 to 18, wherein at least one of the hairpin loop probes comprises complementary, annealed stem loop sequence regions wherein each strand of the stem loop sequence region is capable of being hybridised by a distinct single-stranded hairpin loop sequence of a different hairpin loop probe of the set of four or more hairpin loop nucleic acid probes, thereby forming a branch structure during polymerisation/progression of inter-molecular hybridisation events between hairpin loop probes.

21. A method for the detection of a target nucleic acid in a sample comprising:
a). performing a hybridisation reaction reacting a Target Initiation probe with a sample,
b). performing a hybridisation reaction reacting the product of step a) with a set of Chain Loop probes wherein on binding of the target nucleic acid to the Target Initiation probe the stem region of the Target Initiation probe opens and permits hybridisation to the loop region of the first Chain Loop probe and causes sequential polymerisation of the set of Chain Loop probes to form a polymer or dendrimer capable of generating a signal
c). detecting the presence or absence of a signal that indicates the presence or absence of the target nucleic acid;
**characterised in that**:
the Target Initiation probe in its closed configuration is a hairpin nucleic acid comprising complementary 3' and 5' stem sequences to form a double stranded stem portion and a single stranded target loop portion comprising a sequence complementary to the target nucleic acid;
further **characterised in that** a first Chain Loop probe comprises a hairpin loop structure with a sequence in the loop complementary to the 5' or 3' stem sequence of the Target Initiation probe and a signal generating component;
further **characterised in that** a second Chain Loop probe comprises a hairpin loop structure with a sequence in the loop complementary to the 5' or 3' stem sequence of the first Chain Loop probe and a signal generating component;
further characterised that subsequent Chain Loop probes comprise a hairpin loop structures with a sequence complementary to the 5' or 3' stem sequence of a prior Chain Loop probe and a signal generating component;
wherein on binding of the target nucleic acid to the Target Initiation probe the stem region of the Target Initiation probe opens and permits hybridisation to the loop region of the first Chain Loop probe and causes sequential polymerisation of multiple Chain Loop probes to form a polymer capable of generating a signal; and wherein one of the Chain Loop probes comprises a hairpin loop structure with a further nucleic acid sequence extending from the 5' or 3' stem sequence which hybridises to the target loop of another Chain Loop probe splitting the chain formation into two chains to form a dendrimer capable of generating a signal.

22. A method of claim 21, wherein the reaction of step a) and/or step b) is carried out in the presence of formamide in the hybridising buffer.

23. A method of either of claim 21 or 22, wherein the hybridisation of step a) is carried out at 62.5 °C and/or step b) is carried out at 50 °C.

24. A method of any of claims 21 to 23, for the detection of two or more nucleic acid sequences in a sample wherein step a) comprises two or more sets of Target Initiation probes and step b) comprises one or more sets of Chain Loop probes capable of reporting on the presence or absence of one or more target nucleic acid sequences.

25. A method of any of claims 21 to , wherein the target nucleic acid in the sample is an RNA or a DNA from an infectious agent or a tumour sample selected from clinical, veterinary, food or environmental sources.

## Patentansprüche

1. Kit für die Detektion einer Zielnukleinsäure von Interesse; wobei das Kit Folgendes umfasst:
a) eine Haarnadelzielinitiierungssonde, und
b) einen Satz von mehreren Haarnadelkettenschleifensonden, um ein detektierbares Signal zu verstärken, das eine Signalerzeugungskomponente umfasst, die innerhalb der Kettenschleifensonde enthalten ist;
**dadurch gekennzeichnet, dass**:
die Zielinitiierungssonde in ihrer geschlossenen Konfiguration eine Haarnadelnukleinsäure ist, die eine komplementäre 3'- und 5'-Stammsequenz umfasst, um einen doppelsträngigen Stammabschnitt und einen einsträngigen Zielschleifenabschnitt zu bilden, der eine Sequenz komplementär zu der Zielnukleinsäure umfasst;
ferner **dadurch gekennzeichnet, dass** eine erste Kettenschleifensonde eine Haarnadelschleifenstruktur mit einer Sequenz in der Schleife komplementär zu der 5'- oder 3'-Stammsequenz der Zielinitiierungssonde und eine Signalerzeugungskomponente umfasst;
ferner **dadurch gekennzeichnet, dass** eine zweite Kettenschleifensonde eine Haarnadelschleifenstruktur mit einer Sequenz in der Schleife komplementär zu der 5'- oder 3'-Stammsequenz der ersten Kettenschleifensonde und eine Signalerzeugungskomponente umfasst;
ferner **dadurch gekennzeichnet, dass** nachfolgende Kettenschleifensonden eine Haarnadelschleifenstruktur mit einer Sequenz komplementär zu der 5'- oder 3'-Stammsequenz einer vorherigen Kettenschleifensonde und eine Signalerzeugungskomponente umfassen;
wobei sich bei Bindung der Zielnukleinsäure an die Zielinitiierungssonde die Stammregion der Zielinitiierungssonde öffnet und Hybridisierung mit der Schleifenregion der ersten Kettenschleifensonde ermöglicht und sequentielle Polymerisation von mehreren Kettenschleifensonden bewirkt, um ein Polymer zu bilden, das in der Lage ist, ein Signal zu erzeugen; und wobei eine der Kettenschleifensonden eine Haarnadelschleifenstruktur mit einer weiteren Nukleinsäuresequenz umfasst, die sich von der 5'- oder 3'-Stammsequenz erstreckt, die zu der Zielschleife einer anderen Kettenschleifensonde hybridisiert, wodurch die Kettenbildung in zwei Ketten gespaltet wird, um ein Dendrimer zu bilden, das in der Lage ist, ein Signal zu erzeugen.

2. Kit nach Anspruch 1, wobei die Signalerzeugungskomponente der Kettenschleifensonden eine oder mehrere Gruppen umfasst, die in der Lage sind, ein Signal durch Fluoreszenz oder Chemilumineszenz zu erzeugen.

3. Kit nach einem vorhergehenden Anspruch, wobei die Signalerzeugungskomponente der Kettenschleifensonden ein Förster(Fluoreszenz-)Resonanzenergietransfer(FRET-)Reportergruppenpaar umfasst, sodass in der geschlossenen Position das Akzeptorfluorophor und das Donorfluorophor getrennt sind, um Resonanztransfer und Erzeugung eines detektierbaren Signals zu verhindern.

4. Kit nach einem vorhergehenden Anspruch, wobei die finale Kettenschleifensonde eine Nukleinsäuresequenz in ihrem Stamm umfasst, die komplementär zu einer Sequenz einer vorhergehenden Kettenschleifensonde ist.

5. Kit nach einem vorhergehenden Anspruch, wobei die freie Gibbs-Energie der Bindung der Zielinitiierungssonde und einer oder mehrerer Kettenschleifensonden unter jeweiligen Hybridisierungsbedingungen negativ ist und die Tₘ der Schaftbildung höher als die effektive Hybridisierungstemperatur der Kettenschleifensonden ist.

6. Kit nach einem vorhergehenden Anspruch, wobei die freie Energie (ΔG), die bei jeweiliger Hybridisierung eines beliebigen Satzes von Kettenschleifensonden erzeugt wird, innerhalb eines Bereichs von ΔG = -X +/- 1,5 kcal/mol ist, wobei X -21 kcal/mol bei 50 °C plus 20 % Formamid und 215 mM NaCl entspricht.

7. Kit nach einem vorhergehenden Anspruch, wobei die Kettenschleifensonde eine Zielschleife mit einer einzigartigen Sequenz trägt.

8. Kit nach einem vorhergehenden Anspruch, wobei die 5'- oder die 3'-Stammbildungssequenz der Zielinitiierungssonde und der Kettenschleifensonde thermodynamisch Hybridisierung mit einer komplementären Sequenz gegenüber der Basenpaarung ihrer jeweiligen Haarnadelschleife favorisiert und wobei die freie Gibbs-Energie der Stammbildung jeder Sonde weniger negativ als die freie Gibbs-Energie der Hybridisierung mit einer Zielnukleinsäuresequenz ist.

9. Kit nach Anspruch 3 bis 8, wobei das Reporterpaar ein detektierbares Signal nur erzeugt, wenn eine Kettenschleifensonde (n) an eine Kettenschleifensonde (n-1) bindet und der Hybrid die Donor- und Akzeptorfluorophore nahe genug bringt, um Förster(Fluoreszenz-)Resonanzenergietransfer (FRET) zu ermöglichen, oder damit ein Paar oder eine chemische Vernetzungsgruppen eine chemische Vernetzung bildet.

10. Kit nach Anspruch 2 bis 9, wobei die Kettenschleifensonde eine Reportergruppe umfasst, die an dem 3'-Ende der Sequenz innerhalb ihrer Schleife und an dem 5'-Ende der Stammsequenz gebunden ist, oder umgekehrt, und wobei die Reportergruppe ein detektierbares Signal nur erzeugt, wenn eine Kettenschleifensonde (n) an eine Kettenschleifensonde (n-1) bindet und der Hybrid die Donor- und Akzeptorfluorophore nahe genug bringt, um Förster(Fluoreszenz-)Resonanzenergietransfer (FRET) zu ermöglichen.

11. Kit nach einem vorhergehenden Anspruch, wobei das reporterzeitaufgelöste FRET-Paar wie folgt ist
Europium und ein Fluorophore, das in der Lage ist, durch die Emission von Fluoreszenzlicht, das von Europium emittiert wird, angeregt zu werden, wobei bevorzugt das Europium mit einem Chelatbildner komplexiert ist, wobei optional der Chelatbildner DOTA, EDTA oder ein DOTA- oder EDTA-Analogon ist und das Fluorophor durch den Europium-Emissionspeak bei 620 nm angeregt wird;
Terbium und ein Fluorophor, das in der Lage ist, durch die Emission von Fluoreszenzlicht, das von Terbium emittiert wird, angeregt zu werden, wobei bevorzugt das Terbium mit einem Chelatbildner komplexiert ist, wobei optional der Chelatbildner DOTA, EDTA oder ein DOTA- oder EDTA-Analogon ist und das Fluorophor durch den Terbium-Emissionspeak bei 495 nm angeregt wird; oder
Samarium und ein Fluorophor, das in der Lage ist, durch die Emission von Fluoreszenzlicht, das von Samarium emittiert wird, angeregt zu werden, wobei bevorzugt das Samarium mit einem Chelatbildner komplexiert ist, wobei optional der Chelatbildner DOTA, EDTA oder ein DOTA- oder EDTA-Analogon ist und das Fluorophor durch den Samarium-Emissionspeak bei 350 nm angeregt wird.

12. Kit nach Anspruch 1, wobei Kettenschleifensonden Biotineinheiten als Bindungspunkte für Biotin/Streptavidin-basierte Detektionssysteme tragen.

13. Kit nach einem vorhergehenden Anspruch, umfassend zwei oder mehr Sätze von Zielinitiierungssonden und zwei oder mehr Sätze von Kettenschleifensonden, die in der Lage sind, über die Gegenwart oder Abwesenheit von zwei oder mehr Zielnukleinsäuren zu berichten.

14. Kit nach einem vorhergehenden Anspruch, ferner umfassend eine Einfangsonde; wobei optional die Einfangsonde an ein Partikel gebunden ist, wobei ferner optional das Partikel ein magnetisches Partikel ist.

15. Zusammensetzung, umfassend einen Satz von Sonden mit Sequenzen ausgewählt aus der Gruppe bestehend aus: SEQ ID NOs: 21-25; SEQ ID NOs: 5-10; und SEQ ID NOs: 15-20.

16. Verfahren zum Auswählen von Sequenzen für einen Satz von vier oder mehr Haarnadelschleifennukleinsäuresonden, die in der Lage sind, linear oder exponentiell miteinander in der Gegenwart einer Zielsequenz zu hybridisieren, um ein Kit nach Anspruch 1 zu erzeugen, wobei das Verfahren Folgendes umfasst:
Identifizieren von Kandidatensequenzen für eine erste Haarnadelschleifensonde, eine zweite Haarnadelschleifensonde, eine dritte Haarnadelschleifensonde, eine vierte Haarnadelschleifensonde und optional nachfolgende zusätzliche Haarnadelschleifensonden, wobei jede von der ersten Haarnadelschleifensonde, der zweiten Haarnadelschleifensonde, der dritten Haarnadelschleifensonde, der vierten Haarnadelschleifensonde und der optional nachfolgenden zusätzlichen Haarnadelschleifensonden:
(i) eine Haarnadelstruktur besitzt, wobei die Sonde 5'-terminale und 3'-terminale komplementäre, angelagerte Stammschleifensequenzregionen umfasst, optional umfassend einen terminalen Überhang zwischen 5'- und 3'-Termini, und eine einsträngige Haarnadelschleifensequenz, welche die angelagerten Stammsequenzregionen in einer Lösung verknüpft, der andere Nukleinsäuresequenzen fehlen, wobei die angelagerten Stammsequenzen jeder Haarnadelschleife ausgewählt sind, um einen höheren Wert der freien Gibbs-Energie (ΔG) in Lösung für intramolekulare Stammschleifenstrukturbildung als der ΔG-Wert für intermolekulare Anlagerung zwischen der einsträngigen Haarnadelschleifensequenz und ihrer Zielsequenz in einem separaten Oligonukleotid zu besitzen (d. h. das separate Oligonukleotid ist eine Initiatorzielsequenz oder eine komplementäre Sequenz innerhalb einer Stammschleife einer separaten Haarnadelsonde); und
(ii) eine 5'- oder 3'-terminale erste Fluorophormodifikation und eine zweite Fluorophormodifikation an einer internen Nukleotidrestposition benachbart zu oder innerhalb der einsträngigen Haarnadelschleifensequenz besitzt;
wobei thermodynamische Eigenschaften beinhaltend Werte der freien Gibbs-Energie (ΔG) der Kandidatensequenzen vorhergesagt oder evaluiert werden, um einen Satz von vier oder mehr Haarnadelschleifennukleinsäuresondensequenzen zu identifizieren, wobei:
die einsträngige Haarnadelschleifensequenz der ersten Haarnadelschleifensonde komplementär zu einer Zielsequenz ist, die innerhalb des Satzes von Haarnadelschleifensondensequenzen nicht gefunden wird, wobei die einsträngige Haarnadelschleifensequenz der ersten Haarnadelschleifensonde in der Lage ist, an die Zielsequenz mit einem ΔG-Wert anzulagern, der niedriger als der ΔG-Wert für intramolekulare erste Haarnadelschleifensondenstammschleifenstrukturbildung ist;
die einsträngige Haarnadelschleifensequenz der zweiten Haarnadelschleifensonde komplementär zu zumindest einer Stammregionsequenz der ersten Haarnadelschleifensonde ist und die einsträngige Haarnadelschleifensequenz der zweiten Haarnadelschleifensonde in der Lage ist, an die komplementäre Stammregionsequenz der ersten Haarnadelschleifensonde mit einem ΔG-Wert anzulagern, der niedriger als der ΔG-Wert für intramolekulare zweite Haarnadelschleifensondenstammschleifenstrukturbildung ist;
die einsträngige Haarnadelschleifensequenz der dritten Haarnadelschleifensonde komplementär zu zumindest einer Stammregionsequenz der zweiten Haarnadelschleifensonde ist und die einsträngige Haarnadelschleifensequenz der dritten Haarnadelschleifensonde in der Lage ist, an die komplementäre Stammregionsequenz der zweiten Haarnadelschleifensonde mit einem ΔG-Wert anzulagern, der niedriger als der ΔG-Wert für intramolekulare dritte Haarnadelschleifensondenstammschleifenstrukturbildung ist; und
die einsträngige Haarnadelschleifensequenz der vierten Haarnadelschleifensonde komplementär zu zumindest einer Stammregionsequenz der dritten Haarnadelschleifensonde ist und die einsträngige Haarnadelschleifensequenz der vierten Haarnadelschleifensonde in der Lage ist, an die komplementäre Stammregionsequenz der dritten Haarnadelschleifensonde mit einem ΔG-Wert anzulagern, der niedriger als der ΔG-Wert für intramolekulare vierte Haarnadelschleifensondenstammschleifenstrukturbildung ist,
wodurch Sequenzen für einen Satz von vier oder mehr Haarnadelschleifennukleinsäuresonden ausgewählt werden, die in der Lage sind, linear oder exponentiell miteinander in der Gegenwart einer Zielsequenz zu hybridisieren.

17. Verfahren nach Anspruch 16, wobei jede von der ersten Haarnadelschleifensonde, der zweiten Haarnadelschleifensonde, der dritten Haarnadelschleifensonde, der vierten Haarnadelschleifensonde und den optional nachfolgenden zusätzlichen Haarnadelschleifensonden eine Gesamtlänge von 120 Nukleotiden oder weniger, optional 100 Nukleotiden oder weniger, optional 90 Nukleotiden oder weniger, optional 80 Nukleotiden oder weniger und optional 70 Nukleotiden oder weniger besitzt.

18. Verfahren nach Anspruch 16 oder 17, wobei:
die Haarnadelsequenz der ersten Haarnadelschleifensonde in der Lage ist, an die Zielsequenz mit einem ΔG von zumindest etwa -10 kcal/mol ± 1,5 kcal/mol anzulagern;
die Haarnadelsequenz der zweiten Haarnadelschleifensonde in der Lage ist, an die komplementäre Stammregionsequenz der ersten Haarnadelschleifensonde mit einem ΔG von zumindest etwa -10 kcal/mol ± 1,5 kcal/mol anzulagern;
die Haarnadelsequenz der dritten Haarnadelschleifensonde in der Lage ist, an die komplementäre Stammregionsequenz der zweiten Haarnadelschleifensonde mit einem ΔG von zumindest etwa -10 kcal/mol ± 1,5 kcal/mol anzulagern;
die Haarnadelsequenz der vierten Haarnadelschleifensonde in der Lage ist, an die komplementäre Stammregionsequenz der dritten Haarnadelschleifensonde mit einem ΔG von zumindest etwa -10 kcal/mol ± 1,5 kcal/mol anzulagern; und/oder
die Haarnadelsequenz der (n+1)-ten Haarnadelschleifensonde in der Lage ist, an die komplementäre Stammregionsequenz der n-ten Haarnadelschleifensonde mit einem ΔG von zumindest etwa -10 kcal/mol ± 1,5 kcal/mol anzulagern.

19. Verfahren nach einem der Ansprüche 16 bis 18, ferner umfassend Synthetisieren des Satzes von vier oder mehr Haarnadelschleifennukleinsäuresonden, wodurch Sequenzen für den Satz ausgewählt und hergestellt werden.

20. Verfahren nach einem der Ansprüche 16 bis 19, wobei zumindest eine von den Haarnadelschleifensonden komplementäre, angelagerte Stammschleifensequenzregionen umfasst, wobei jeder Strang der Stammschleifensequenzregion in der Lage ist, durch eine separate einsträngige Haarnadelschleifensequenz einer anderen Haarnadelschleifensonde des Satzes von vier oder mehr Haarnadelschleifennukleinsäuresonden hybridisiert zu werden, wodurch eine Zweigstruktur während Polymerisation/Fortschritt von intermolekularen Hybridisierungsereignissen zwischen Haarnadelschleifensonden gebildet wird.

21. Verfahren für die Detektion einer Zielnukleinsäure in einer Probe, umfassend:
a). Durchführen einer Hybridisierungsreaktion, die eine Zielinitiierungssonde mit einer Probe reagiert,
b). Durchführen einer Hybridisierungsreaktion, die das Produkt von Schritt a) mit einem Satz von Kettenschleifensonden reagiert, wobei sich bei Bindung der Zielnukleinsäure an die Zielinitiierungssonde die Stammregion der Zielinitiierungssonde öffnet und Hybridisierung zu der Schleifenregion der ersten Kettenschleifensonde ermöglicht und sequentielle Polymerisation des Satzes von Kettenschleifensonden bewirkt, um ein Polymer oder Dendrimer zu bilden, das in der Lage ist, ein Signal zu erzeugen,
c). Detektieren der Gegenwart oder Abwesenheit eines Signals, das die Gegenwart oder Abwesenheit der Zielnukleinsäure angibt;
**dadurch gekennzeichnet, dass**:
die Zielinitiierungssonde in ihrer geschlossenen Konfiguration eine Haarnadelnukleinsäure ist, die komplementäre 3'- und 5'-Stammsequenzen umfasst, um einen doppelsträngigen Stammabschnitt und einen einsträngigen Zielschleifenabschnitt zu bilden, der eine Sequenz komplementär zu der Zielnukleinsäure umfasst;
ferner **dadurch gekennzeichnet, dass** eine erste Kettenschleifensonde eine Haarnadelschleifenstruktur mit einer Sequenz in der Schleife komplementär zu der 5'- oder 3'-Stammsequenz der Zielinitiierungssonde und eine Signalerzeugungskomponente umfasst;
ferner **dadurch gekennzeichnet, dass** eine zweite Kettenschleifensonde eine Haarnadelschleifenstruktur mit einer Sequenz in der Schleife komplementär zu der 5'- oder 3'-Stammsequenz der ersten Kettenschleifensonde und eine Signalerzeugungskomponente umfasst;
ferner **dadurch gekennzeichnet, dass** nachfolgende Kettenschleifensonden eine Haarnadelschleifenstrukturen mit einer Sequenz komplementär zu der 5'- oder 3'-Stammsequenz einer vorherigen Kettenschleifensonde und eine Signalerzeugungskomponente umfassen;
wobei sich bei Bindung der Zielnukleinsäure an die Zielinitiierungssonde die Stammregion der Zielinitiierungssonde öffnet und Hybridisierung zu der Schleifenregion der ersten Kettenschleifensonde ermöglicht und sequentielle Polymerisation von mehreren Kettenschleifensonden bewirkt, um ein Polymer zu bilden, das in der Lage ist, ein Signal zu erzeugen; und wobei eine der Kettenschleifensonden eine Haarnadelschleifenstruktur mit einer weiteren Nukleinsäuresequenz umfasst, die sich von der 5'- oder 3'-Stammsequenz erstreckt, die zu der Zielschleife einer anderen Kettenschleifensonde hybridisiert, wodurch die Kettenbildung in zwei Ketten gespaltet wird, um ein Dendrimer zu bilden, das in der Lage ist, ein Signal zu erzeugen.

22. Verfahren nach Anspruch 21, wobei die Reaktion von Schritt a) und/oder Schritt b) in der Gegenwart von Formamid in dem Hybridisierungspuffer durchgeführt wird.

23. Verfahren nach einem von Anspruch 21 oder 22, wobei die Hybridisierung von Schritt a) bei 62,5 °C durchgeführt wird und/oder Schritt b) bei 50 °C durchgeführt wird.

24. Verfahren nach einem der Ansprüche 21 bis 23 für die Detektion von zwei oder mehr Nukleinsäuresequenzen in einer Probe, wobei Schritt a) zwei oder mehr Sätze von Zielinitiierungssonden umfasst und Schritt b) einen oder mehrere Sätze von Kettenschleifensonden umfasst, die in der Lage sind, über die Gegenwart oder Abwesenheit von einer oder mehreren Zielnukleinsäuresequenzen zu berichten.

25. Verfahren nach einem der Ansprüche 21 bis 24, wobei die Zielnukleinsäure in der Probe eine RNA oder eine DNA von einem infektiösen Mittel oder eine Tumorprobe ausgewählt aus klinischen, tiermedizinischen, Lebensmittel- oder Umweltquellen ist.

## Revendications

1. Kit permettant la détection d'un acide nucléique cible d'intérêt ; le kit comprenant :
a) une sonde d'initiation de cible en épingle à cheveux, et
b) un ensemble de multiples sondes en boucle de chaîne en épingle à cheveux pour amplifier un signal détectable comprenant un composant générateur de signal contenu à l'intérieur de la sonde en boucle de chaîne ;
**caractérisé en ce que** :
la sonde d'initiation de cible, dans sa configuration fermée, est un acide nucléique en épingle à cheveux comprenant des séquences de tige complémentaires 3' et 5' pour former une partie tige double brin et une partie boucle de cible simple brin comprenant une séquence complémentaire à l'acide nucléique cible ;
en outre **caractérisé en ce qu'**une première sonde en boucle de chaîne comprend une structure en boucle en épingle à cheveux présentant, dans la boucle, une séquence complémentaire à la séquence de tige 5' ou 3' de la sonde d'initiation de cible et un composant générateur de signal ;
en outre **caractérisé en ce qu'**une seconde sonde en boucle de chaîne comprend une structure en boucle en épingle à cheveux présentant, dans la boucle, une séquence complémentaire à la séquence de tige 5' ou 3' de la première sonde en boucle de chaîne et un composant générateur de signal ;
en outre **caractérisé en ce que** des sondes en boucle de chaîne suivantes comprennent des structures en boucle en épingle à cheveux présentant une séquence complémentaire à la séquence de tige 5' ou 3' d'une sonde en boucle de chaîne précédente et un composant générateur de signal ;
dans lequel, lors de la liaison de l'acide nucléique cible à la sonde d'initiation de cible, la région de tige de la sonde d'initiation de cible s'ouvre et permet l'hybridation à la région de boucle de la première sonde en boucle de chaîne et provoque la polymérisation séquentielle de multiples sondes en boucle de chaîne pour former un polymère capable de générer un signal ; et dans lequel l'une des sondes en boucle de chaîne comprend une structure en boucle en épingle à cheveux avec une séquence d'acide nucléique supplémentaire s'étendant à partir de la séquence de tige 5' ou 3' qui s'hybride à la boucle de cible d'une autre sonde en boucle de chaîne divisant la formation de chaîne en deux chaînes pour former un dendrimère capable de générer un signal.

2. Kit selon la revendication 1, dans lequel le composant générateur de signal des sondes en boucle de chaîne comprend un ou plusieurs groupes capables de générer un signal par fluorescence ou chimiluminescence.

3. Kit selon une quelconque revendication précédente, dans lequel le composant générateur de signal des sondes en boucle de chaîne comprend une paire de groupes rapporteurs de transfert d'énergie par résonance (FRET) de Förster (fluorescence), de sorte que, dans la position fermée, le fluorophore accepteur et le fluorophore donneur soient séparés pour empêcher le transfert par résonance et la génération d'un signal détectable.

4. Kit selon une quelconque revendication précédente, dans lequel la sonde en boucle de chaîne finale comprend une séquence d'acide nucléique dans sa tige qui est complémentaire à une séquence d'une sonde en boucle de chaîne précédente.

5. Kit selon une quelconque revendication précédente, dans lequel l'énergie libre de Gibbs de liaison de la sonde d'initiation de cible et d'une ou plusieurs sondes en boucle de chaîne est négative dans des conditions d'hybridation respectives et la Tₘ de la formation de tige est supérieure à la température d'hybridation effective des sondes en boucle de chaîne.

6. Kit d'une quelconque revendication précédente, dans lequel l'énergie libre (ΔG) générée lors de l'hybridation respective de tout ensemble de sonde en boucle de chaîne est comprise dans une plage de ΔG = -X +/- 1,5 kcal/mol, où X est égal à -21 kcal/mol à 50 °C plus 20 % de formamide et 215 mM de NaCl.

7. Kit d'une quelconque revendication précédente, dans lequel une sonde en boucle de chaîne porte une boucle de cible présentant une séquence unique.

8. Kit d'une quelconque revendication précédente, dans lequel la séquence de formation de tige 5' ou 3' de la sonde d'initiation de cible et de la sonde en boucle de chaîne favorise thermodynamiquement l'hybridation avec une séquence complémentaire par rapport à l'appariement de base de sa boucle en épingle à cheveux respective, et dans lequel l'énergie libre de Gibbs de formation de tige de chaque sonde est moins négative que l'énergie libre de Gibbs d'hybridation avec une séquence d'acide nucléique cible.

9. Kit selon les revendications 3 à 8, dans lequel la paire de rapporteurs génère un signal détectable uniquement lorsqu'une sonde en boucle de chaîne (n) se lie à une sonde en boucle de chaîne (n-1) et que l'hybridation rapproche suffisamment les fluorophores donneur et accepteur pour permettre un transfert d'énergie par résonance (FRET) de Förster (fluorescence) ou pour qu'une paire ou un groupe de réticulation chimique forme une réticulation chimique.

10. Kit selon les revendications 2 à 9, dans lequel la sonde en boucle de chaîne comprend un groupe rapporteur lié à l'extrémité 3' de la séquence à l'intérieur de sa boucle et à l'extrémité 5' de la séquence de tige, ou inversement, et dans lequel le groupe rapporteur génère un signal détectable uniquement lorsqu'une sonde en boucle de chaîne (n) se lie à une sonde en boucle de chaîne (n-1) et que l'hybridation rapproche suffisamment les fluorophores donneur et accepteur pour permettre le transfert d'énergie par résonance (FRET) de Förster (fluorescence).

11. Kit d'une quelconque revendication précédente, dans lequel la paire FRET à résolution temporelle de rapporteur est constituée d'europium et d'un fluorophore capable d'être excité par l'émission de lumière fluorescente émise par l'europium, de préférence dans lequel l'europium est complexé à un agent chélateur, éventuellement dans lequel l'agent chélateur est DOTA, EDTA ou un analogue de DOTA ou d'EDTA, et le fluorophore est excité par le pic d'émission de l'europium à 620 nm ;
de terbium et d'un fluorophore capable d'être excité par l'émission de lumière fluorescente émise par le terbium, de préférence dans lequel le terbium est complexé à un agent chélateur, éventuellement dans lequel l'agent chélateur est DOTA, EDTA ou un analogue de DOTA ou d'EDTA, et le fluorophore est excité par le pic d'émission du terbium à 495 nm ; ou
de samarium et d'un fluorophore capable d'être excité par l'émission de lumière fluorescente émise par le samarium, de préférence dans lequel le samarium est complexé à un agent chélateur, éventuellement dans lequel l'agent chélateur est DOTA, EDTA ou un analogue de DOTA ou d'EDTA, et le fluorophore est excité par le pic d'émission du samarium à 350 nm.

12. Kit selon la revendication 1, dans lequel des sondes en boucle de chaîne portent des fractions de biotine en tant que points de liaison pour des systèmes de détection à base de biotine/streptavidine.

13. Kit selon une quelconque revendication précédente, comprenant deux ensembles ou plus de sondes d'initiation de cible et deux ensembles ou plus de sondes en boucle de chaîne capables de signaler la présence ou l'absence de deux acides nucléiques cibles ou plus.

14. Kit selon une quelconque revendication précédente, comprenant en outre une sonde de capture ; éventuellement dans lequel la sonde de capture est liée à une particule, dans lequel éventuellement en outre la particule est une particule magnétique.

15. Composition comprenant un ensemble de sondes présentant des séquences choisies dans le groupe constitué de : SEQ ID N° : 21-25 ; SEQ ID N° : 5-10 ; et SEQ ID N° : 15-20.

16. Procédé permettant la sélection de séquences pour un ensemble de quatre sondes d'acide nucléique en boucle en épingle à cheveux ou plus capables de s'hybrider de manière linéaire ou exponentielle les unes aux autres en présence d'une séquence cible pour générer un kit selon la revendication 1, le procédé comprenant :
l'identification de séquences candidates pour une première sonde en boucle en épingle à cheveux, une deuxième sonde en boucle en épingle à cheveux, une troisième sonde en boucle en épingle à cheveux, une quatrième sonde en boucle en épingle à cheveux et des sondes en boucle en épingle à cheveux supplémentaires éventuellement successives, où chacune de la première sonde en boucle en épingle à cheveux, de la deuxième sonde en boucle en épingle à cheveux, de la troisième sonde en boucle en épingle à cheveux, de la quatrième sonde en boucle en épingle à cheveux et des sondes en boucle en épingle à cheveux supplémentaires éventuellement successives :
(i) possède une structure en épingle à cheveux, ladite sonde comprenant des régions de séquence de tige-boucle hybridées et complémentaires aux extrémités 5' et 3', comprenant éventuellement une extrémité sortante terminale entre les extrémités 5' et 3', et une séquence de boucle en épingle à cheveux simple brin qui relie les régions de séquence de tige hybridées dans une solution dépourvue d'autres séquences d'acides nucléiques, lesdites séquences de tige hybridées de chaque boucle en épingle à cheveux étant choisies, de manière à présenter, en solution, une valeur d'énergie libre Gibbs (ΔG) plus élevée pour la formation de structure de tige-boucle intramoléculaire que la valeur ΔG pour l'hybridation intermoléculaire entre la séquence de boucle en épingle à cheveux simple brin et sa séquence cible dans un oligonucléotide distinct (c'est-à-dire, l'oligonucléotide distinct étant une séquence cible initiatrice ou une séquence complémentaire à l'intérieur d'une tige-boucle d'une sonde en épingle à cheveux distincte) ; et
(ii) possède une première modification par fluorophore à l'extrémité 5' ou 3' et une seconde modification par fluorophore à une position de résidu nucléotidique interne adjacente à ou à l'intérieur de la séquence de boucle en épingle à cheveux simple brin ;
dans lequel les propriétés thermodynamiques, y compris les valeurs d'énergie libre de Gibbs (ΔG) des séquences candidates, sont prédites ou évaluées pour identifier un ensemble de quatre séquences de sonde d'acide nucléique en boucle en épingle à cheveux ou plus, dans lequel :
la séquence de boucle en épingle à cheveux simple brin de la première sonde en boucle en épingle à cheveux est complémentaire d'une séquence cible non trouvée dans l'ensemble de séquences de sonde en boucle en épingle à cheveux, dans lequel la séquence de boucle en épingle à cheveux simple brin de la première sonde en boucle en épingle à cheveux est capable de s'hybrider à la séquence cible avec une valeur ΔG qui est inférieure à la valeur ΔG pour la formation de structure de tige-boucle de première sonde en boucle en épingle à cheveux intramoléculaire ;
la séquence de boucle en épingle à cheveux simple brin de la deuxième sonde en boucle en épingle à cheveux est complémentaire d'au moins une séquence de région de tige de la première sonde en boucle en épingle à cheveux et la séquence de boucle en épingle à cheveux simple brin de la deuxième sonde en boucle en épingle à cheveux est capable de s'hybrider à la séquence de région de tige complémentaire de la première sonde en boucle en épingle à cheveux avec une valeur ΔG qui est inférieure à la valeur ΔG pour la formation de structure de tige-boucle de deuxième sonde en boucle en épingle à cheveux intramoléculaire ;
la séquence de boucle en épingle à cheveux simple brin de la troisième sonde en boucle en épingle à cheveux est complémentaire d'au moins une séquence de région de tige de la deuxième sonde en boucle en épingle à cheveux et la séquence de boucle en épingle à cheveux simple brin de la troisième sonde en boucle en épingle à cheveux est capable de s'hybrider à la séquence de région de tige complémentaire de la deuxième sonde en boucle en épingle à cheveux avec une valeur ΔG qui est inférieure à la valeur ΔG pour la formation de structure de tige-boucle de troisième sonde en boucle en épingle à cheveux intramoléculaire ; et
la séquence de boucle en épingle à cheveux simple brin de la quatrième sonde en boucle en épingle à cheveux est complémentaire d'au moins une séquence de région de tige de la troisième sonde en boucle en épingle à cheveux et la séquence de boucle en épingle à cheveux simple brin de la quatrième sonde en boucle en épingle à cheveux est capable de s'hybrider à la séquence de région de tige complémentaire de la troisième sonde en boucle en épingle à cheveux avec une valeur ΔG qui est inférieure à la valeur ΔG pour la formation de structure de tige-boucle de quatrième sonde en boucle en épingle à cheveux intramoléculaire,
ainsi, la sélection de séquences pour un ensemble de quatre sondes d'acide nucléique en boucle en épingle à cheveux ou plus capables de s'hybrider de manière linéaire ou exponentielle les unes aux autres en présence d'une séquence cible.

17. Procédé de la revendication 16, dans lequel chacune de la première sonde en boucle en épingle à cheveux, de la deuxième sonde en boucle en épingle à cheveux, de la troisième sonde en boucle en épingle à cheveux, de la quatrième sonde en boucle en épingle à cheveux et des sondes en boucle en épingle à cheveux supplémentaires éventuellement successives possède une longueur totale inférieure ou égale à 120 nucléotides, éventuellement inférieure ou égale à 100 nucléotides, éventuellement inférieure ou égale à 90 nucléotides, éventuellement inférieure ou égale à 80 nucléotides, et éventuellement inférieure ou égale à 70 nucléotides.

18. Procédé de la revendication 16 ou 17, dans lequel :
la séquence d'épingle à cheveux de la première sonde en boucle en épingle à cheveux est capable de s'hybrider à la séquence cible avec une ΔG d'au moins environ - 10 kcal/mol ± 1,5 kcal/mol ;
la séquence d'épingle à cheveux de la deuxième sonde en boucle en épingle à cheveux est capable de s'hybrider à la séquence de région de tige complémentaire de la première sonde en boucle en épingle à cheveux avec une ΔG d'au moins environ -10 kcal/mol ± 1,5 kcal/mol ;
la séquence d'épingle à cheveux de la troisième sonde en boucle en épingle à cheveux est capable de s'hybrider à la séquence de région de tige complémentaire de la deuxième sonde en boucle en épingle à cheveux avec une ΔG d'au moins environ -10 kcal/mol ± 1,5 kcal/mol ;
la séquence d'épingle à cheveux de la quatrième sonde en boucle en épingle à cheveux est capable de s'hybrider à la séquence de région de tige complémentaire de la troisième sonde en boucle en épingle à cheveux avec une ΔG d'au moins environ -10 kcal/mol ± 1,5 kcal/mol ; et/ou
la séquence d'épingle à cheveux de la (n+1)^{ième} sonde en boucle en épingle à cheveux est capable de s'hybrider à la séquence de région de tige complémentaire de la n^{ième} sonde en boucle en épingle à cheveux avec une ΔG d'au moins environ -10 kcal/mol ± 1,5 kcal/mol.

19. Procédé de l'une quelconque des revendications 16 à 18, comprenant en outre la synthèse de l'ensemble de quatre sondes d'acide nucléique en boucle en épingle à cheveux ou plus, sélectionnant et préparant ainsi des séquences pour l'ensemble.

20. Procédé de l'une quelconque des revendications 16 à 19, dans lequel au moins l'une des sondes en boucle en épingle à cheveux comprend des régions de séquence de tige-boucle hybridées et complémentaires, dans lequel chaque brin de la région de séquence de tige-boucle est capable d'être hybridé par une séquence de boucle en épingle à cheveux simple brin distincte d'une sonde en boucle en épingle à cheveux différente de l'ensemble de quatre sondes d'acide nucléique en boucle en épingle à cheveux ou plus, formant ainsi une structure ramifiée pendant la polymérisation/progression d'événements d'hybridation intermoléculaire entre des sondes en boucle en épingle à cheveux.

21. Procédé permettant la détection d'un acide nucléique cible dans un échantillon comprenant :
a). la réalisation d'une réaction d'hybridation en faisant réagir une sonde d'initiation de cible avec un échantillon,
b). la réalisation d'une réaction d'hybridation en faisant réagir le produit de l'étape a) avec un ensemble de sondes en boucle de chaîne, où lors de la liaison de l'acide nucléique cible à la sonde d'initiation de cible, la région de tige de la sonde d'initiation de cible s'ouvre et permet l'hybridation à la région de boucle de la première sonde en boucle de chaîne et provoque la polymérisation séquentielle de l'ensemble de sondes en boucle de chaîne pour former un polymère ou un dendrimère capable de générer un signal
c). la détection de la présence ou de l'absence d'un signal indiquant la présence ou l'absence de l'acide nucléique cible ;
**caractérisé en ce que** :
la sonde d'initiation de cible, dans sa configuration fermée, est un acide nucléique en épingle à cheveux comprenant des séquences de tige complémentaires 3' et 5' pour former une partie tige double brin et une partie boucle de cible simple brin comprenant une séquence complémentaire à l'acide nucléique cible ;
en outre **caractérisé en ce qu'**une première sonde en boucle de chaîne comprend une structure en boucle en épingle à cheveux présentant, dans la boucle, une séquence complémentaire à la séquence de tige 5' ou 3' de la sonde d'initiation de cible et un composant générateur de signal ;
en outre **caractérisé en ce qu'**une seconde sonde en boucle de chaîne comprend une structure en boucle en épingle à cheveux présentant, dans la boucle, une séquence complémentaire à la séquence de tige 5' ou 3' de la première sonde en boucle de chaîne et un composant générateur de signal ;
en outre **caractérisé en ce que** des sondes en boucle de chaîne suivantes comprennent des structures en boucle en épingle à cheveux présentant une séquence complémentaire à la séquence de tige 5' ou 3' d'une sonde en boucle de chaîne précédente et un composant générateur de signal ;
dans lequel, lors de la liaison de l'acide nucléique cible à la sonde d'initiation de cible, la région de tige de la sonde d'initiation de cible s'ouvre et permet l'hybridation à la région de boucle de la première sonde en boucle de chaîne et provoque la polymérisation séquentielle de multiples sondes en boucle de chaîne pour former un polymère capable de générer un signal ; et dans lequel l'une des sondes en boucle de chaîne comprend une structure en boucle en épingle à cheveux avec une séquence d'acide nucléique supplémentaire s'étendant à partir de la séquence de tige 5' ou 3' qui s'hybride à la boucle de cible d'une autre sonde en boucle de chaîne divisant la formation de chaîne en deux chaînes pour former un dendrimère capable de générer un signal.

22. Procédé de la revendication 21, dans lequel la réaction de l'étape a) et/ou de l'étape b) est réalisée en présence de formamide dans le tampon d'hybridation.

23. Procédé de l'une quelconque des revendications 21 ou 22, dans lequel l'hybridation de l'étape a) est réalisée à 62,5 °C et/ou l'étape b) est réalisée à 50 °C.

24. Procédé de l'une quelconque des revendications 21 à 23, permettant la détection de deux séquences d'acides nucléiques ou plus dans un échantillon, dans lequel l'étape a) comprend deux ensembles ou plus de sondes d'initiation de cible et l'étape b) comprend un ou plusieurs ensembles de sondes en boucle de chaîne capables de signaler la présence ou l'absence d'une ou plusieurs séquences d'acides nucléiques cibles.

25. Procédé de l'une quelconque des revendications 21 à 24, dans lequel l'acide nucléique cible dans l'échantillon est un ARN ou un ADN provenant d'un agent infectieux ou d'un échantillon de tumeur choisi parmi des sources cliniques, vétérinaires, alimentaires ou environnementales.
